# DEMANDE DE BREVET EUROPEEN

(11) **EP 2 189 148 A2**
(43) Date de publication de la demande: **26.05.2010**
(21) Numéro de dépôt: 09176118.9
(22) Date de dépôt: 16.11.2009
(51) Int. Cl.: A61K 8/02, A61K 8/04, A61K 8/06, A61K 8/31, A61K 8/81, A61K 8/91, A61Q 1/10, A61Q 5/06, C08L 23/00

(54) **Composition de coloration des fibres kératiniques comprenant un polymère supramoléculaire à base de polyalcène, un pigment et un solvant volatil**

(30) Priorité: 24.11.2008 FR 0857936
(71) Demandeur: L'Oréal, 75008 Paris (FR)
(72) Inventeur: Brun, Gaëlle, 75011, Paris (FR); Chodorowski-Kimmes, Sandrine, 60300, Senlis (FR)
(74) Mandataire: Prevel, Estelle Nicole

(57) **Abrégé**

La présente invention a pour objet une composition de coloration des fibres kératiniques comprenant un ou plusieurs polymères supramoléculaires à base de polyalcène, un ou plusieurs pigments et un ou plusieurs solvants volatils, le rapport pondéral polymères supramoléculaires / pigments étant supérieur à 0,25, ainsi qu'un procédé de coloration des fibres kératiniques mettant en oeuvre cette composition.

La présente invention permet d'obtenir sur les fibres kératiniques des gainages colorés permettant de conserver des cheveux individualisés tout en préservant les qualités physiques de la fibre.

## Description

La présente invention a pour objet une composition de coloration des fibres kératiniques, et en particulier des fibres kératiniques humaines telles que les cheveux ou les cils, comprenant un ou plusieurs polymères supramoléculaires à base de polyalcène, un ou plusieurs pigments et un ou plusieurs solvants volatils, le rapport pondéral polymères supramoléculaires / pigments étant supérieur à 0,25.

Dans le domaine de la coloration des fibres kératiniques, il est déjà connu de colorer des fibres kératiniques par différentes techniques à partir de colorants directs pour des colorations non permanentes ou de précurseurs de colorants pour des colorations permanentes.

La coloration non permanente ou coloration directe consiste à teindre les fibres kératiniques avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, xanthénique, acridinique, azinique, triarylméthane ou des colorants naturels.

Certains de ces colorants peuvent être utilisés dans des conditions éclaircissantes ce qui permet d'obtenir des colorations visibles sur des cheveux foncés.

Il est aussi connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs sous l'action d'un agent oxydant vont former dans le cheveu une ou plusieurs espèces colorées.

La variété des molécules mises en jeu au niveau des bases d'oxydation et des coupleurs, permet l'obtention d'une riche palette de couleurs et les colorations qui en résultent sont en général permanentes, puissantes, et résistantes aux agents extérieurs, notamment à la lumière, aux intempéries, aux lavages, à la transpiration et aux frottements.

Pour être visibles sur cheveux foncés, ces deux techniques de coloration nécessitent une décoloration préalable ou simultanée des fibres kératiniques. Cette étape de décoloration mise en oeuvre avec un agent oxydant tel que le peroxyde d'hydrogène ou des persels entraîne une dégradation non négligeable des fibres kératiniques ce qui altère leurs propriétés cosmétiques. Les cheveux ont alors tendance à devenir rêches, plus difficilement démêlables et plus fragiles.

Une autre méthode de coloration consiste à utiliser des pigments. En effet, l'utilisation de pigment à la surface des fibres kératiniques permet en général d'obtenir des colorations visibles sur cheveux foncés puisque le pigment en surface masque la couleur naturelle de la fibre. L'utilisation de pigment pour colorer des fibres kératiniques est par exemple décrite dans la demande de brevet FR 2 741 530, qui préconise l'utilisation pour la coloration temporaire des fibres kératiniques d'une composition comprenant au moins une dispersion de particules de polymère filmogène comportant au moins une fonction acide et au moins un pigment dispersé dans la phase continue de ladite dispersion.

Les colorations obtenues par ce mode de coloration présentent l'inconvénient de s'éliminer dès le premier shampooing.

Il est par ailleurs connu de la demande de brevet FR 2 907 678 d'effectuer des gainages colorés des cheveux à partir d'une composition comprenant un copolymère bloc polysiloxane / polyurée et un pigment. Cependant, avec une telle composition, les gainages obtenus ne sont pas toujours très homogènes et l'individualisation des cheveux n'est pas toujours très bonne.

Il est également connu du brevet EP 1 392 222 d'utiliser une composition cosmétique pour le soin et / ou le traitement des matières kératiniques comprenant un polymère supramoléculaire comportant un squelette polymérique et au moins deux groupements capables de former au moins trois liaisons hydrogène, et du brevet EP 1 435 900 d'utiliser une composition capillaire comprenant un polymère supramoléculaire comportant un squelette polymérique et au moins deux groupements capables de former au moins trois liaisons hydrogène et un agent tensio-actif ou un agent de conditionnement des cheveux.

Ainsi, le but de la présente invention est de fournir une composition de coloration des fibres kératiniques, et en particulier des fibres kératiniques humaines telles que les cheveux ou les cils, qui permet d'obtenir des gainages colorés rémanents aux shampoings et aux diverses agressions que peuvent subir les cheveux sans dégradation des fibres kératiniques et tout en conservant des cheveux parfaitement individualisés.

Ce but est atteint avec la présente invention qui a pour objet une composition de coloration des fibres kératiniques, et en particulier des fibres kératiniques humaines telles que les cheveux ou les cils, comprenant un ou plusieurs polymères supramoléculaires à base de polyalcène, un ou plusieurs pigments et un ou plusieurs solvants volatils, le rapport pondéral polymères supramoléculaires / pigments étant supérieur à 0,25, de préférence allant de 0,3 à 20, encore plus préférentiellement de 0,4 à 10, mieux de 0,5 à 5.

La composition conforme à la présente invention permet d'obtenir sur les fibres kératiniques des gainages colorés permettant d'obtenir une coloration visible sur tous types de cheveux, notamment sur les cheveux foncés, de façon rémanente aux shampooings tout en préservant les qualités physiques de la fibre kératinique. Un tel gainage est en particulier résistant aux agressions extérieures que peuvent subir les cheveux telles que le brushing et la transpiration. Il permet en particulier d'obtenir un dépôt lisse et homogène. Par ailleurs, on a constaté de façon surprenante que les cheveux restaient parfaitement individualisés, pouvaient être coiffés sans problème.

On entend par cheveux individualisés des cheveux qui après application de la composition et séchage ne sont pas collés (ou sont tous séparés les uns des autres) entre eux et ne forment donc pas des amas de cheveux, le gainage étant formé autour de pratiquement chaque cheveu.

La présente invention a également pour objet un procédé de coloration des fibres kératiniques, et en particulier des fibres kératiniques humaines telles que les cheveux ou les cils, mettant en oeuvre cette composition.

La présente invention a aussi pour objet l'utilisation de cette composition pour l'obtention de gainage coloré sur les cheveux.

Dans ce qui suit, sauf indication contraire, les bornes des intervalles indiqués sont comprises dans l'invention.

### Polymères supramoléculaires à base de polyalcène

Au sens de la présente invention, on entend par polymère supramoléculaire à base de polyalcène un polymère comportant dans sa structure au moins une partie polyalcène et au moins une partie comportant au moins un groupe capable de former au moins 3 liaisons H, de préférence 4 liaisons H.

Le polyalcène est choisi de préférence parmi les poly(éthylènebutylène), les polybutadiènes et les polyisoprènes.

Les polymères supramoléculaires de l'invention peuvent être notamment issus de la condensation d'au moins un polymère polyalcène fonctionnalisé par au moins un groupe réactif, avec au moins un greffon fonctionnalisé par au moins un groupe réactif susceptible de réagir avec le ou les groupes réactifs du polymère poly(alcène) fonctionnalisé, le dit greffon étant porteur d'au moins un groupe capable de former au moins 3 liaisons H, de préférence au moins 4 liaisons H.

De préférence, le polymère polyalcène fonctionnalisé est de formule A :

HX-R-X'H

XH et X'H étant des groupes réactifs, avec X et X', identiques ou différents, choisis parmi O, S, NH, ou NRₐ, Rₐ représentant un groupe alkyle en C₁-C₆ de préférence, X et / ou X' désignent O ; encore plus préférentiellement, X et X' désignent O ;
R représente un homo ou un copolymère issu d'un ou plusieurs alcènes mono ou polyinsaturés en C₂-C₁₀, et de préférence en C₂-C₄; R représente de préférence un poly(éthylènebutylène), un polybutadiène ou un polyisoprène.

Les poly(éthylènebutylène) sont des copolymères de butène 1 et d'éthylène. On peut les schématiser par l'enchaînement de motifs de structures:

[-CH₂-CH₂-]

et

[-CH₂CH(CH₂-CH₃)]

Les polybutadiènes peuvent être des 1,4-polybutadiènes ou des 1,2-polybutabiènes qui peuvent être respectivement schématiser par les enchaînements de motifs suivants :

[-CH₂-CH=CH-CH₂-] (polybutadiènes 1,4)

[-CH₂-CH(CH=CH₂)-] (polybutadiènes 1,2)

De preference, pour les polybutadiènes, il s'agit de polybutadiènes 1,2.

La fonctionnalisation se fait de préférence en bout de chaînes. On parle alors de polymères téléchéliques. Les groupes de fonctionnalisation peuvent être fixés au polymère polyalcène via des linkers, de préférence des groupes alkylène en C₁-C₄ linéaires ou ramifiés.

Ces poly(alcènes) peuvent être hydrogénés pour éviter les risques de réticulations.

Ils peuvent comporter dans leur structure d'autres motifs issus d'autres monomères. Comme comonomères, on peut en particulier citer le styrène.

Les polydiènes, de préférence hydrogénés, à extrémités hydroxyles et les polyoléfines à extrémités hydroxyles sont des squelettes polymériques préférés selon l'invention.

Ces polydiènes à extrémités hydroxyles sont définis par exemple dans le brevet FR 2 782 723 d'ELF ATOCHEM. Ils sont choisis dans le groupe comprenant les homo et copolymères de polybutadiène, de polyisoprène et de poly(1,3-pentadiène). Ce sont des oligomères de masse moléculaire moyenne en nombre inférieure à 7000, et de préférence de 1000 à 5000, présentant une fonctionnalité en extrémités hydroxyles de 1,8 à 3, et de préférence voisine de 2.

On citera en particulier les polybutadiènes hydroxylés commercialisés par la société ELF ATOCHEM sous les marques POLY BD R-45HT et POLY BD R-20 LM, qui seront utilisés de préférence hydrogénés. On peut aussi citer les (1,2-polybutadiènes) hydrogénés di OH tels que le GI3000 de Mn = 2600-3200 et le GI2000 de Mn = 1800-2200 commercialisés par la société Nisso.

On peut également utiliser des polyoléfines, homopolymères ou copolymères, à extrémités α,ω hydroxyles tels que :
- les oligomères de polyisobutylène à extrémités α,ω hydroxyles ;
- les copolymères commercialisés par la société Mitsubishi sous la marque POLYTAIL avec, en particulier, ceux répondant à la formule :

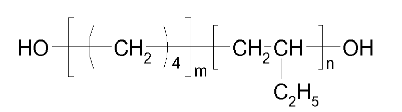

Les polymères supramoléculaires de la présente invention possèdent dans leur structure au moins un greffon étant porteur d'au moins un groupe capable de former au moins 3 liaisons H, de préférence au moins 4 liaisons H.

Ces groupes capables de former au moins 3 liaisons H peuvent comprendre par exemple au moins 3 groupements fonctionnels, de préférence au moins 4, choisis parmi :

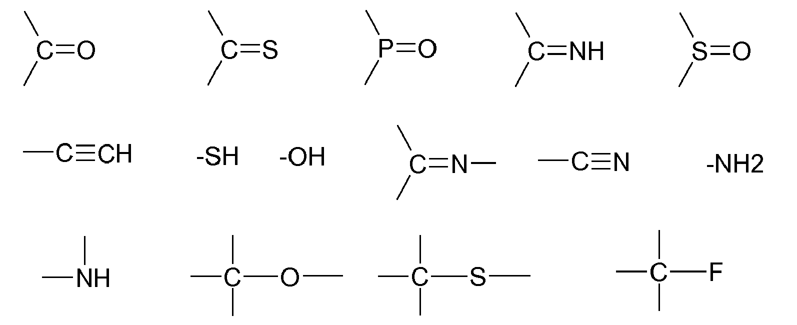

Ces groupements fonctionnels peuvent être classés en deux catégories :
- les groupements fonctionnels donneurs de liaisons H tels que les groupements : 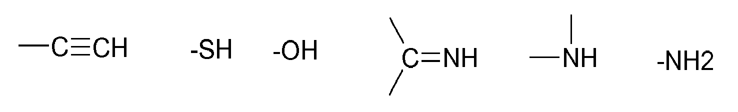
- et les groupements fonctionnels accepteurs de liaisons H tels que les groupements :

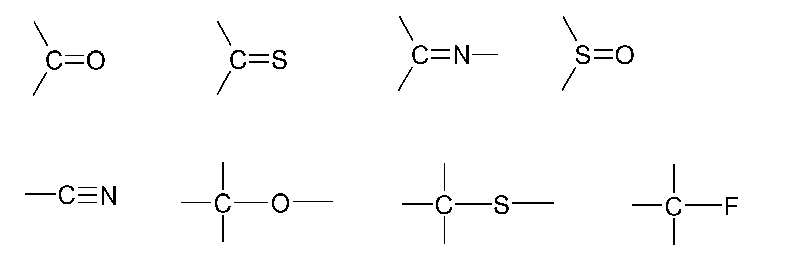

Les groupes capables de former au moins 3 liaisons H forment un élément structural de base comportant au moins 3 groupements, de préférence au moins 4 groupements fonctionnels, et plus préférentiellement 4 groupements fonctionnels, capables d'établir des liaisons H. Les éléments structuraux de base capables d'établir 3 ou 4 liaisons H peuvent être schématisés de la manière suivante : 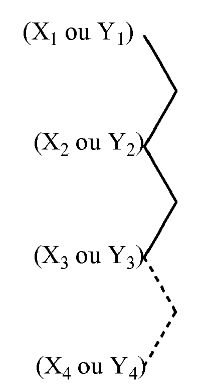 où Xᵢ (i entier naturel) est un groupement fonctionnel accepteur de liaisons H et Yᵢ est un groupement fonctionnel donneur de liaisons H.

Ainsi, chaque élément structural doit pouvoir établir des liaisons H avec un ou plusieurs éléments structuraux partenaires, identiques (c'est-à-dire auto-complémentaires) ou différents, de telle sorte que chaque appariement de deux éléments structuraux partenaires se fasse par formation d'au moins trois liaisons H, de préférence au moins quatre liaisons H, et plus préférentiellement 4 liaisons H.
Un accepteur de protons X s'appariera avec un donneur de protons Y.
Plusieurs possibilités sont offertes, par exemple appariement de :
XXXX avec YYYY ;
XXXY avec YYYX ;
XXYX avec YYXY ;
XYYX avec YXXY ;
XXYY avec YYXX auto-complémentaire ou non ;
XYXY avec YXYX auto-complémentaire ou non.

De préférence, les groupes peuvent établir 4 liaisons H avec un groupe partenaire identique (ou autocomplémentaire) parmi lesquelles 2 liaisons donneur (par exemple NH) et 2 liaisons accepteur (par exemple CO et -C=N-).

De préférence, les groupes capables de former au moins 3 liaisons H comportent des cycles à 5 ou 6 atomes (cycles aromatiques ou hétérocycles insaturés) très souvent constitués d'atomes de C et / ou N et avec des doubles liaisons conjuguées pour stabiliser et diriger les interactions H.

De préférence encore, les groupes capables de former au moins 3 liaisons H sont engagés dans des cycles à 6 atomes comprenant des C et / ou N et avec des doubles liaisons conjuguées pour stabiliser et diriger les interactions H.

Selon un mode de réalisation particulier de l'invention, les groupes capables de former 3 ou 4 liaisons H sont choisis parmi les familles suivantes, étant entendu que toutes les formes tautomériques sont inclues :
- (i) les aminopyrimidones de formule : 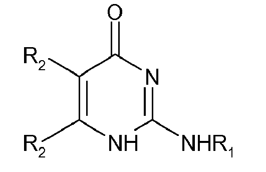
- (ii) les ureïdopyrimidones de formule : 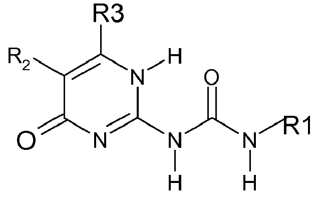
- (iii) les acylaminopyridines et notamment :
- les monoacylaminopyridines de structure : 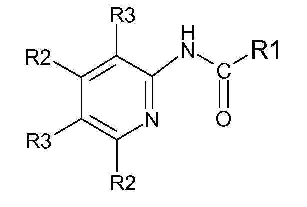
- les di(acylamino)pyridines et plus particulièrement les 2,6-di(acylamino)pyridines de structure : 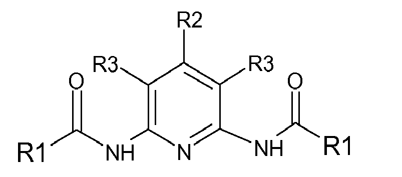
- (iv) les aminopyrimidines, et notamment :
- les composés aminopyrimidines : 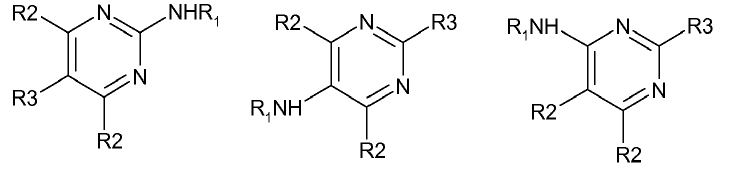
- les composés diaminopyrimidines de structure : 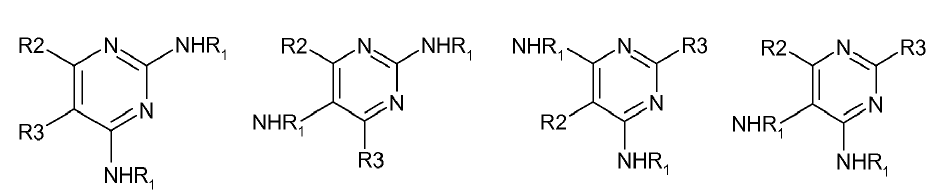
- les composés triaminopyrimidines ;
- (v) les uréïdotriazines, et notamment les mono-, di- et tri-uréïdotriazines, et en particulier les uréïdoaminotriazines de structure : 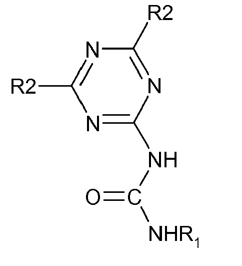
- (vi) les (acylamino)triazines, et notamment les mono-, di- et tri-acylamino triazines, éventuellement amino (mono-, di- ou tri-amino) et en particulier :
- les di(acylamino)triazines de structure : 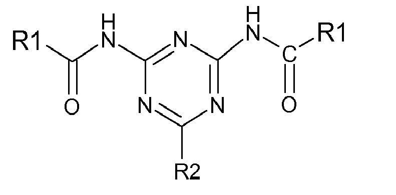
- les acylamino, amino-triazines, (mono ou di- acylamino, et mono- ou di-amino) et notamment les composés de structure : 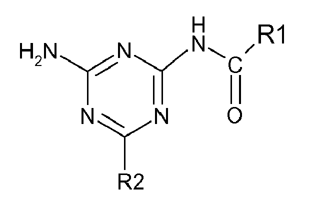
- les acylaminotriazines de structure : 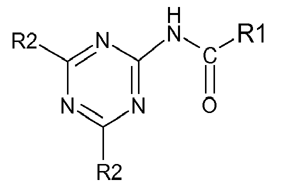
- les tri-acylamino triazines ;
- (vii) les aminotriazines et notamment :
- les monoaminotriazines ;
- les 2,6-diamino-s-triazines de structure : 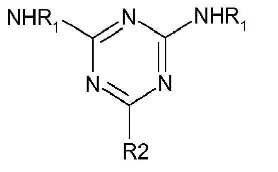
- les composés triamino-s-triazines de structure : 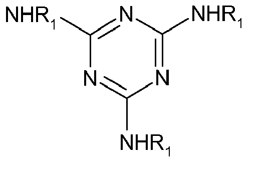
- (viii) les acylaminotriazoles de structure : 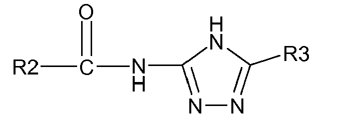
- (ix) les composés de la famille de l'acide urazoyl benzoïque de structure : 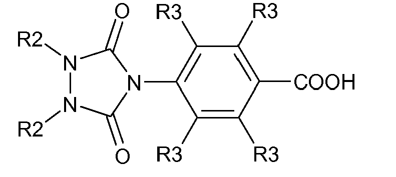
- (x) les phtalhydrazides de structure : 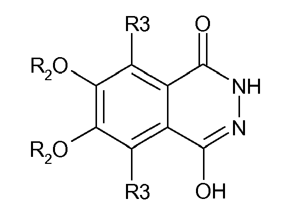
- (xi) les uraciles de structure : 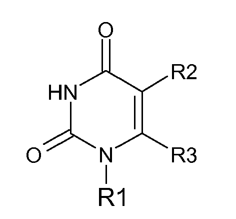
- (xii) les thymines de structure : 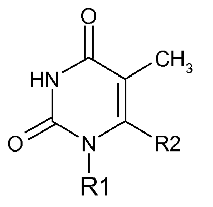
- (xiii) les succinimides de structure : 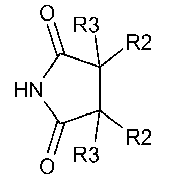
- (xiv) les glutarimides de structure : 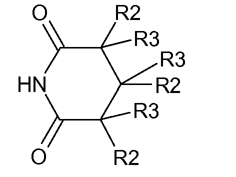
- (xv) les composés de la famille de l'acide cyanurique de structure : 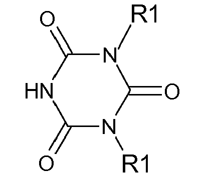
- (xvi) les maléimides : 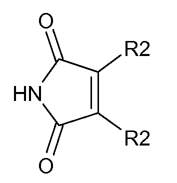
- (xvii) les composés de la famille de l'acide barbiturique, de structure : 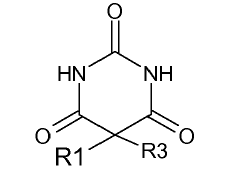
- (xviii) les composés de structure : 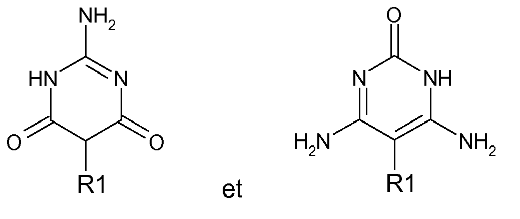
- (xix) les composés de la famille de l'acide triméllitique, de formule : 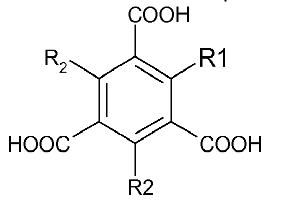
- (xx) les uréïdopyridines, notamment les mono- ou di-uréïdopyridines, et en particulier ceux de formule : 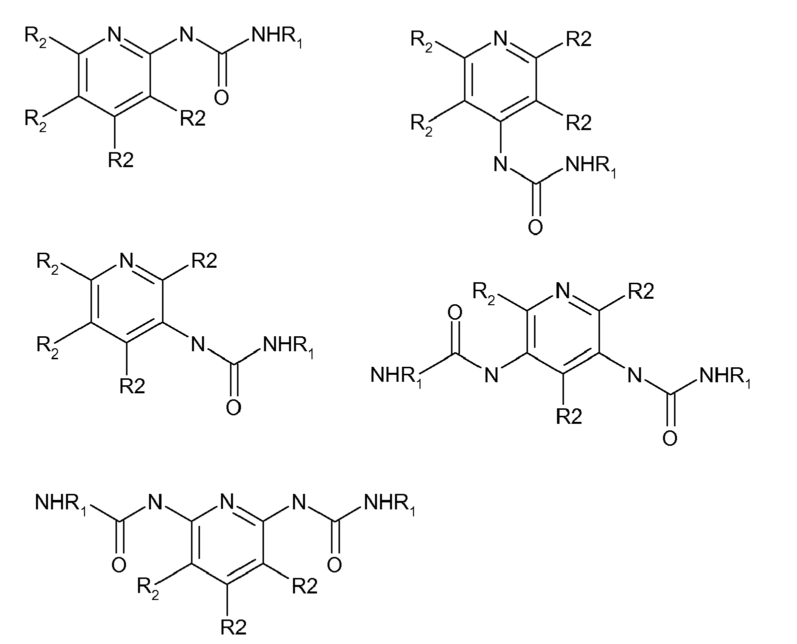
- (xxi) les carbamoylpyridines, de formule : 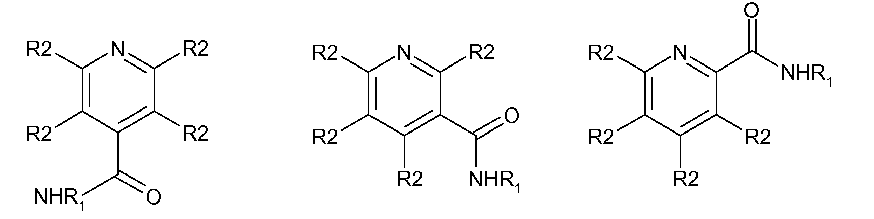
- (xxii) les adénines de formule : 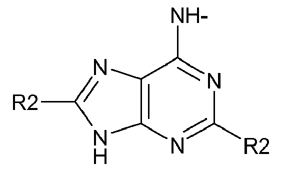
- (xxiii) les guanines de formule : 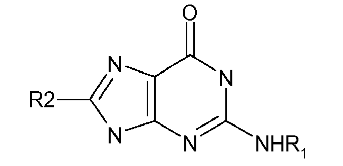
- (xxiv) les cytidines de formule :

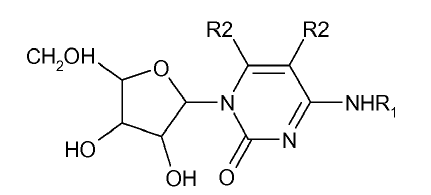

Dans l'ensemble de ces formules, la signification des radicaux est la suivante :
- (a) les radicaux R₁, identiques ou différents, représentent une liaison simple, un atome d'hydrogène, un atome d'halogène et / ou un groupe carboné (notamment alkyle) monovalent, linéaire, ramifié ou cyclique, en C₁-C₆₀₀₀, saturé ou non, éventuellement aromatique, pouvant contenir un ou plusieurs hétéroatomes tels que O, S, N, P, CI, Br, CI, Br, F ; ou une combinaison de ces significations.
   Le radical R₁ peut notamment être un groupe cycloalkyle en C₄-C₁₂ ; un groupe alkyle linéaire ou ramifié en C₁-C₃₀ ou un groupe aryle en C₄-C₁₂ ; éventuellement substitués par une fonction amino, ester et / ou hydroxy.
   Le radical R₁ peut être aussi un groupement: C₄Hg; phényle; 1,4-nitrophényle; 1,2-éthylène; 1,6-héxylène; 1,4-butylène; 1,6-(2,4,4-triméthylhexylène); 1,4-(4-méthylpentylène) ; 1,5-(5-méthylhexylène) ; 1,6-(6-méthylheptylène); 1,5-(2,2,5-triméthylhexylène) ; 1,7-(3,7-diméthyloctylène); - isophorone- ; 4,4'-méthylène biscyclohexylène; tolylène ; 2-méthyl-1,3-phénylène ; 4-méthyl-1,3-phénylène ; 4,4-biphénylèneméthylène; et de préférence : -isophorone- ; -(CH₂)₂-; -(CH₂)₆-; -CH₂CH(CH₃)-CH₂-C(CH₃)₂-CH₂-CH₂; 4,4'-méthylène biscyclohexylène; 2-méthyl-1,3-phénylène.
   Encore plus préférentiellement, R₁ est une liaison simple.
- (b) les radicaux R₂, identiques ou différents au sein d'une même formule, représentent une liaison simple, un atome d'hydrogène, un atome d'halogène (-Br, -Cl, -F), un radical -OH, -N(R)₂ (avec R étant H ou un radical alkyle, linéaire ou ramifié en C₁-C₁₂, de préférence en C₁-C₄, et mieux un radical méthyle ou éthyle) ; ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F ; ou une combinaison de ces significations.
   Les radicaux R₂ peuvent notamment être H, CN, NH₂ ou bien :
   - un groupe alkyle en C₁-C₃₀ ;
   - un groupe cycloalkyle en C₄-C₁₂ ;
   - un groupe aryle en C₄-C₁₂ ;
   - un groupe aryl(C₄-C₁₂)alkyle en C₁-C₃₀ ;
   - un groupe alcoxy en C₁-C₄ ;
   - un groupe arylalcoxy, en particulier un groupe aryl(C₁-C₄)alcoxy ;
   - un hétérocycle en C₄-C₁₂ ;
   - un groupe thioalcoxy ;
   - un groupe sulfoxy ;
   ou leurs mélanges, ces groupes étant éventuellement substitués par une fonction amino, ester et / ou hydroxy.
   De préférence, R₂ représente H, CH₃, C₁₃H₂₇, C₇H₁₅ ou phényle.
- (c) les radicaux R₃, identiques ou différents au sein d'une même formule, représentent un atome d'hydrogène ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F ; ou une combinaison de ces significations.

Le radical R₃ peut notamment être un groupe cycloalkyle en C₄-C₁₂ ; un groupe alkyle linéaire ou ramifié en C₁-C₃₀ ou un groupe aryle en C₄-C₁₂ ; éventuellement substitués par une fonction amino, ester et / ou hydroxy. De préférence, le radical R₃ représente un radical méthyle.

Dans l'ensemble de ces formules, il est bien entendu qu'au moins un, notamment un ou deux, des groupes R₁ et / ou R₂ est une liaison simple constituant le point d'accroche du groupe capable de former au moins 3 liaisons H sur le reste du greffon.

De préférence, ledit point d'accroche est porté par R₁ et / ou R₂ et de préférence, il est porté par R₁.

Les groupes capables de former au moins 3 liaisons H peuvent notamment être choisis parmi :
(a) les groupes capables de former au moins 3 liaisons H complémentaires et identiques c'est-à-dire auto-complémentaires, et notamment :
   - les aminopyrimidones, les uréïdopyrimidones,
   - les composés de la famille de l'acide triméllitique, ou de l'acide urazoyl benzoïque,
   - les acylaminopyridines, les uréïdopyridines, les carbamoylpyridines,
   - les acylaminotriazines, les uréidotriazines et notamment les uréïdoaminotriazines, les diamino-triazines,
   - les acylaminotriazoles,
   - les phtalhydrazides,
   - les composés de formule : 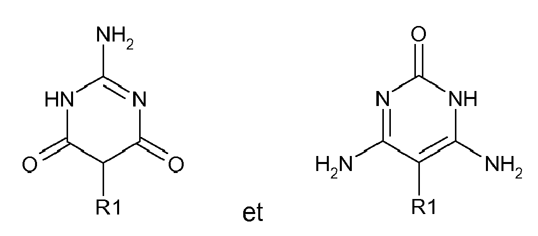
   dans lesquelles R₁ est un atome d'hydrogène ou un groupe hydrocarboné monovalent, linéaire, ramifié ou cyclique, en C₁-C₆₀₀₀, saturé ou non, éventuellement aromatique, pouvant contenir un ou plusieurs hétéroatomes tels que O, S, N, P, F.
(b) les groupes capables de former au moins 3 liaisons H complémentaires mais différents, et notamment :
   - adénine complémentaire de guanine,
   - cytidine complémentaire de thymine,
   - triamino-s-triazine complémentaire de uracile ou de succinimide ou de glutarimide ou d'acide cyanurique ou de thymine ou de maléimide ou de (di)aminopyrimidine ou d'acide barbiturique,
   - acylamino-amino-s-triazine complémentaire de uracile ou de succinimide ou de glutarimide ou d'acide cyanurique ou de thymine ou de maléimide ou de (di)aminopyrimidine ou d'acide barbiturique.

De manière préférée, les groupes capables de former au moins 3 liaisons H sont choisis parmi les groupes capables d'établir au moins trois liaisons H avec eux-mêmes (auto-complémentaire), notamment au moins quatre liaisons H avec eux-mêmes. Parmi ces groupes, on peut en particulier citer :
- les uréïdopyrimidones ;
- les uréïdopyridines, les carbamoylpyridines ;
- les acylamino-s-triazines et notamment les acyl-diamino-s-triazines ;
- les uréidotriazines ;
- les phtalhydrazides ;
- les composés de formule : 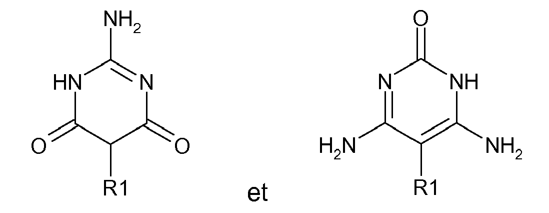
dans lesquels les radicaux R₁, R₂ et R₃ ont les significations données ci-dessus, en particulier les significations données en préférence.

Encore mieux, on peut citer à titre d'exemples préférés de groupes capables de former au moins 3 liaisons H les groupes dérivés des uréidopyrimidones, et en particulier de la 2-uréïdopyrimidone ou de la 6-méthyl, 2-uréïdopyrimidone.

Le reste du greffon est constitué d'un linker L porteur d'au moins un groupe réactif capable de réagir avec le ou les groupe du poly(alcène) fonctionnalisé.

Ce groupe réactif peut être par exemple un groupe carboxy ou un groupe isocyanate. De préférence, il s'agit d'un groupe -N=C=O ou -N=C=S, et encore plus préférentiellement d'un groupe -N=C=O (isocyanate).

De préférence, le linker L est un groupement: phénylène; 1,4-nitrophényle; 1,2-éthylène; 1,6-héxylène; 1,4-butylène; 1,6-(2,4,4-triméthylhexylène); 1,4-(4-méthylpentylène) ; 1,5-(5-méthylhexylène) ; 1,6-(6-méthylheptylène); 1,5-(2,2,5-triméthylhexylène) ; 1,7-(3,7-diméthyloctylène) ; - isophorone- ; 4,4'-méthylène biscyclohexylène; tolylène ; 2-méthyl-1,3-phénylène ; 4-méthyl-1,3-phénylène ; 4,4-biphénylèneméthylène ;
et de préférence : -isophorone- ; -(CH₂)₂- ; -(CH₂)₆- ; -CH₂CH(CH₃)-CH₂-C(CH₃)₂-CH₂-CH₂ ; 4,4'-méthylène biscyclohexylène ; 2-méthyl-1,3-phénylène.

Dans une version particulièrement préférée de l'invention, les greffons sont de formule (B) : 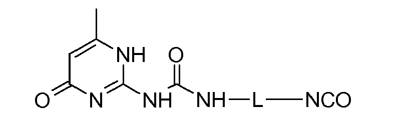 L ayant la même signification que ci-dessus.

Encore plus préférentiellement, le polymère supramoléculaire de l'invention est de formule (C) : 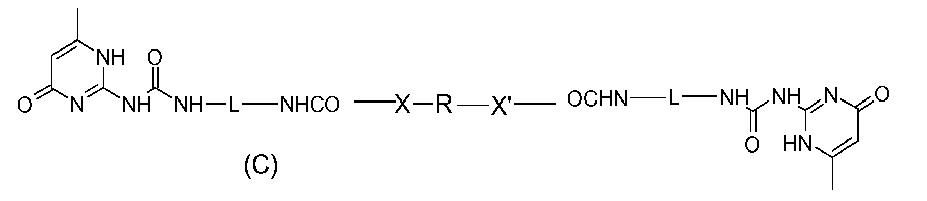 R, X, X', L ayant les significations indiquées précédemment.

De préférence, dans la formule (C), X et X' désignent un atome d'oxygène.

Le ou les polymères supramoléculaires à base de polyalcène de l'invention peuvent aussi être obtenus à partir d'un polymère (A1) comportant une partie polyalcène, le dit polymère étant fonctionnalisé par au moins un groupe réactif (B1), par condensation avec au moins une molécule (A3) comportant au moins un groupe réactif (B2), la dite molécule étant telle qu'après réaction des groupes (B1) et (B2) il y ait formation d'une entité capable de former au moins 3 liaisons H, de préférence au moins 4 liaisons H.

De préférence, ces entités sont de structures (i) à (xxiv) telles que définies précédemment avec R₁ désignant une simple liaison.

Le polymère (A1) peut notamment résulter de l'action sur un polymère polyalcène de formule A telle que défine ci avant de composés (A2) comportant deux groupes réactifs (B'2) capables de réagir avec les groupes fonctionnalisés du polyalcène.

Ces groupes réactifs peuvent être par exemple des groupes carboxy ou des groupes isocyanate. De préférence, il s'agit de groupes -N=C=O ou -N=C=S, et encore plus préférentiellement de groupes -N=C=O (isocyanate).

De préférence, les groupes B2 sont identiques aux groupes B'2.

De préférence, les composés (A2) sont de structure (C') suivante :

B'2-L'-B'2 (C')

le linker L' ayant les mêmes significations que L défini précédemment.

Dans une version particulièrement préférée de l'invention, les polymères A1 sont de formule (C1) :

CON-L-NCO-X-R-X'-CON-L-NCO (C1)

dans laquelle L, X, X' et R ont les mêmes significations que précédemment.

De préférence, la molécule (A3) est la 6-méthylisocytosine de formule :

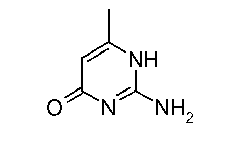

Dans la pratique, le ou les polymères supramoléculaires selon l'invention peuvent être préparés par les procédés usuellement employés par l'homme du métier pour former une liaison uréthanne, entre les fonctions OH libres d'un polyalcène polyhydroxylé et les fonctions isocyanates portées par le groupe de jonction. A titre d'illustration, un procédé général de préparation consiste à :
- s'assurer que le polymère à fonctionnaliser ne comporte pas d'eau résiduelle ;
- chauffer le polymère comportant au moins deux fonctions réactives, notamment OH, à une température pouvant être comprise entre 60 °C et 140 °C. L'indice d'hydroxyle du polymère pourra faire servir de référence afin de mesurer l'état d'avancement de la réaction ;
- ajouter directement le greffon portant les fonctions réactives, notamment isocyanate ;
- agiter le mélange, sous atmosphère contrôlée, à une température de l'ordre de 90-130 °C; pendant 1 à 24 heures ;
- suivre par spectroscopie infrarouge, la disparition de la bande caractéristique des isocyanates (compris entre 2500 et 2800 cm⁻¹) de manière à arrêter la réaction à la disparition totale du pic, puis à laisser revenir à température ambiante le produit final ;
- la réaction pourra aussi être suivi par des dosages des fonctions hydroxyles ;
- un ajout d'éthanol pourra être éventuellement effectué afin de s'assurer de la disparition totale de fonctions isocyanate résiduelles ;
- Le mélange pourra être filtré si nécessaire.

La réaction peut être effectuée en présence d'un solvant, notamment la méthyltétrahydrofurane, le tétrahydrofurane, le toluène ou l'acétate de butyle, ou encore le propylène carbonate.

Il est également possible d'ajouter un catalyseur conventionnel de la formation de liaison uréthane. A titre d'exemple, on peut citer le dilaurate dibutyle étain.

Le composé peut au final être lavé et séché, voire purifié, selon les connaissances générales de l'homme du métier.

Selon un autre mode de réalisation, la réaction peut comporter les étapes suivantes :
(i) Fonctionnalisation du polymère polyalcène P polyhydroxylé préalablement séché par un diisocyanate selon le schéma réactionnel suivant :

   OH - P-OH (1éq) + NCO-X-NCO (1éq) → CN-X-NH-(O)CO- P-OC(O)-NH-X-NCO

   Le diisocyanate peut être éventuellement en excès par rapport au polymère. Cette première étape peut être faite en présence de solvant, à une température comprise entre 20 °C et 100 °C.
   Cette première étape peut être suivie par une période d'agitation, sous atmosphère contrôlée pendant une période allant de 1 heure à 24 heures. Le mélange peut être éventuellement chauffé.
   L'état d'avancement de cette première étape peut être suivi par un dosage des fonctions hydroxyles.
   puis
(ii) réaction du pré-polymère obtenu dans l'étape (i) avec la 6-méthylisocytosine :

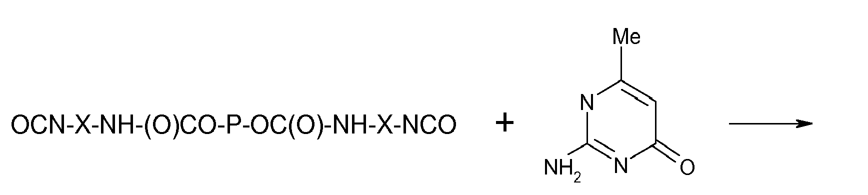

Cette deuxième étape peut éventuellement se faire en présence d'un co-solvant comme le toluène, l'acétate de butyle ou encore le propylène carbonate. Le mélange réactionnel peut être chauffé entre 80 °C et 140 °C pendant un temps variant entre 1 heure et 24 heures.

La présence d'un catalyseur peut favoriser l'obtention du produit final désiré. Nous pouvons citer par exemple l'utilsiation du dilaurate dibutyle étain.

La réaction peut être suivie par spectroscopie infra-rouge, en suivant la disparition du pic caractéristique de l'isocyanate entre 2200 et 2300 cm⁻¹.

A la fin de la réaction, de l'éthanol peut être additionné au milieu réactionnel afin de neutraliser les fonctions isocyanates résiduelles. Le mélange réactionnel peut être éventuellement filtré. Pour les besoins d'applications, le polymère pourra être directement strippé dans un solvant cosmétique.

Le ou les polymères supramoléculaires à base de de polyalcène peuvent être présents dans la composition en une teneur allant de 0,1 % à 40 % en poids, de préférence allant de 0,1 % à 30 % en poids, plus préférentiellement allant de 0,5 % à 20 % en poids, et encore plus préférentiellement allant de 1 % à 15 % en poids par rapport au poids total de la composition.

### Pigments

La composition comprend un ou plusieurs pigments. Une telle composition permet d'obtenir des gainages colorés et rémanents, et ceci sans dégradation des fibres kératiniques.

Par pigment, on entend tous les pigments apportant de la couleur aux matières kératiniques. Leur solubilité dans l'eau à 25 °C et à pression atmosphérique (760 mmHg) est inférieure à 0,05 % en poids, et de préférence inférieure à 0,01 %.

Les pigments qui peuvent être utilisés sont notamment choisis parmi les pigments organiques et / ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments peuvent par exemple être choisis parmi les pigments minéraux, les pigments organiques, les laques, les pigments à effets spéciaux tels que les nacres ou les paillettes, et leurs mélanges.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de fer ou de chrome, le violet de manganèse, le bleu outremer, l'hydrate de chrome, le bleu ferrique et l'oxyde de titane.

Le pigment peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Le pigment organique peut aussi être une laque. Par laque, on entend les colorants adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants, on peut citer le carmin de cochenille. On peut également citer les colorants connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments colorés qui procurent une teinte uniforme opaque, semi-transparente ou transparente classique.

Il existe plusieurs types de pigments à effets spéciaux, ceux à faible indice de réfraction tels que les pigments fluorescents, photochromes ou thermochromes, et ceux à plus fort indice de réfraction tels que les nacres ou les paillettes.

A titre d'exemples de pigments à effets spéciaux, on peut citer les pigments nacrés tels que le mica titane recouvert avec un oxyde de fer, le mica recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona), par la société Eckart sous la dénomination Prestige Bronze et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) et par la société Eckart sous la dénomination Prestige Copper ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), Dark Blue (117324) (Colorona), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

La variété des pigments qui peuvent être utilisés dans la présente invention permet d'obtenir une riche palette de couleurs, ainsi que des effets optiques particuliers tels que des effets métalliques, interférentiels.

La taille du pigment utilisé dans la composition cosmétique selon la présente invention est généralement comprise entre 10 nm et 200 µm, de préférence entre 20 nm et 80 µm, et plus préférentiellement entre 30 nm et 50 µm.

Les pigments peuvent être dispersés dans le produit grâce à un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en C₈ à C₂₀ et de polyol comme le glycérol, la diglycérine, tel que le stéarate d'acide poly(12-hydroxystéarique) de poids moléculaire d'environ 750 g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

Les pigments utilisés dans la composition cosmétique selon l'invention peuvent être traités en surface par un agent organique.

Ainsi les pigments préalablement traités en surface utiles dans le cadre de l'invention sont des pigments qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans la composition cosmétique selon l'invention peuvent aussi avoir été traités par un mélange de ces composés et / ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments.
Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments traités en surface, de préférence de 0,5 à 30 % en poids, et encore plus préférentiellement de 1 à 10 % en poids.

De préférence, les traitements en surface des pigments sont choisis parmi les traitements suivants :
- un traitement PEG-Silicone comme le traitement de surface AQ commercialisé par LCW;
- un traitement Chitosane comme le traitement de surface CTS commercialisé par LCW;
- un traitement Triéthoxycaprylylsilane comme le traitement de surface AS commercialisé par LCW ;
- un traitement Méthicone comme le traitement de surface SI commercialisé par LCW ;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAl commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

La composition conforme à la présente invention peut de plus comprendre un ou plusieurs pigments non traités en surface.

Selon un mode de réalisation particulier de l'invention, le ou les pigments sont des pigments minéraux.

Selon un autre mode particulier de l'invention, le ou les pigments sont choisis parmi les nacres.

La quantité de pigments peut varier de 0,5 % jusqu'à 40 %, et de préférence de 1 à 20 %.

La composition de l'invention peut contenir d'autres espèces colorées ou colorantes telle que des colorants directs hydrophiles ou hydrophobes ou des précurseurs de colorants.

### Solvants volatils

La composition conforme à l'invention comprend un ou plusieurs solvants volatils.

Dans le cadre de l'invention, on entend par solvant volatil, un composé liquide à la température ambiante (20 °C) et à la pression atmosphérique présentant une pression de vapeur à 20 °C supérieure à 0,1 mmHg, et de préférence comprise entre 0,1 et 300 mmHg, encore plus préférentiellement entre 0,5 et 200 mmHg.

Ce solvant volatil peut être de l'eau, un solvant organique non siliconé, un solvant organique siliconé ou leurs mélanges. A titre de solvant organique non siliconé volatil, on peut citer:
- les alcanols volatils en C₁-C₄ tels que l'éthanol, l'isopropanol;
- les alcanes volatils en C₅-C₇ tels que le n-pentane, l'hexane, le cyclopentane, le 2,3-diméthylbutane, le 2,2-diméthylbutane, le 2-méthylpentane, le 3-méthylpentane ;
- les esters d'acides en C₁-C₂₀ liquides et d'alcools en C₁-C₈ volatils tels que l'acétate de méthyle, l'acétate de n-butyle, l'acétate d'éthyle, l'acétate de propyle, l'acétate d'isopentyle, le 3-éthoxypopionate d'éthyle;
- les cétones liquides à température ambiante et volatiles telles que la méthyléthylcétone, la méthylisobutylcétone, la diisobutylcétone, l'isophorone, la cyclohexanone, l'acétone ;
- les éthers volatils tels que le diméthoxyméthane, le diéthoxyéthane, le diéthyléther ;
- les éthers de glycol volatils comme le 2-butoxyéthanol, le butyle diglycol, le monoméhyléther de diéthylène glycol, le n-butyléther de propylène glycol, l'acétate de monométhyl éther de propylène glycol ;
- les huiles hydrocarbonées volatiles telles que les huiles volatiles hydrocarbonées ayant de 8 à 16 atomes de carbone et leurs mélanges, et notamment les alcanes ramifiés en C₈-C₁₆ comme les iso-alcanes (appelées aussi isoparaffines) en C₈-C₁₆, l'isododécane, l'isodécane, et par exemple les huiles vendues sous les noms commerciaux d'Isopars ou de Permetyls, et leurs mélanges. On peut aussi citer les neopentanoate d'isohexyle ou d'isodecyle ;
- les perfluoroalcanes volatils en C₄-C₁₀ tels que dodecafluoropentane, le tetradecafluorohexane, le decafluoropentane ;
- les perfluorocycloalkyles volatils tels que le perfluorométhylcyclopentane, le 1,3-perfluorodiméthylcyclohexane et le perfluorodecaline, vendus respectivement sous les dénominations de "Flutec PC1®", "Flutec PC3®" et "Flutec PC6®" par la Société F2 Chemicals, ainsi que le perfluorodiméthylcyclobutane et la perfluoromorpholine ;
- les composés fluoroalkyles ou hétérofluoroalkyles volatils répondant à la formule suivante :

   CH₃-(CH₂)ₙ-[Z]ₜ-X-CF₃
dans laquelle t est 0 ou 1 ; n est 0, 1, 2 ou 3 ; X est un radical perfluoroalkyle divalent, linéaire ou ramifié, ayant de 2 à 5 atomes de carbone, et Z représente O, S, ou NR, R étant hydrogène, un radical -(CH₂)ₙ-CH₃ ou -(CF₂)ₘ-CF₃, m étant 2, 3, 4 ou 5.

Parmi les composés fluoroalkyles ou hétérofluoroalkyles volatils, on peut notamment citer le méthoxynonafluorobutane vendu sous la dénomination de "MSX 4518®", "HFE-7100®" par la Société 3M et l'éthoxynonafluorobutane vendu sous la dénomination de "HFE-7200®" par la Société 3M.

De préférence, le solvant est choisi de telle manière que son point d'ébullition soit inférieur à 200 °C.

Selon un mode de réalisation particulier, le solvant organique non siliconé est choisi parmi l'éthanol, l'isopropanol, l'acétone, l'isododécane.

A titre de solvant siliconé volatil, on peut citer les composés siliconés à faible viscosité choisi parmi les silicones linéaires ou cycliques ayant de 2 à 7 atomes de silicium, ces silicones comportant éventuellement des groupes alkyle ou alkoxy ayant de 1 à 10 atomes de carbone, par exemple l'octaméthylcyclotétrasiloxane, le décaméthylcyclopentasiloxane, le dodécaméthylcyclohexasiloxane, l'heptaméthylhexyltri-siloxane, l'heptaméthyléthyltrisiloxane, l'octaméthyltrisiloxane, le décaméthyltétrasiloxane, et leurs mélanges. Selon un mode de réalisation particulier, le composé siliconé est choisi parmi le cyclopentadiméthylsiloxane, le dodécaméthylcyclohexasiloxane, l'octaméthyltrisiloxane et le décaméthyltétrasiloxane.

A titre d'exemple on peut citer la décaméthylcyclopentasiloxane commercialisée sous le nom DC-245 par la société Dow Corning, l'octaméthyltrisiloxane commercialisée sous le nom DC-200 Fluid 1 cst par la société Dow Corning et le décaméthyltétrasiloxane commercialisée sous le nom DC-200 Fluid 1.5 cst par la société Dow Corning.

Selon un mode de réalisation particulier de l'invention, le ou les solvants volatils sont choisis parmi l'eau, l'éthanol, l'isopropanol, l'acétone, l'isododécane, décaméthylcyclopentasiloxane, l'octaméthyltrisiloxane et le décaméthyltétrasiloxane et leurs mélanges.

Le solvant volatil peut être présent dans la composition utile dans le procédé de l'invention en une teneur allant de 0,1 % à 95 % en poids, par rapport au poids total de la composition, de préférence allant de 1 % à 90 % en poids, et préférentiellement allant de 5 % à 90 % en poids.

### Autres solvants

La composition de l'invention peut de plus contenir d'autres solvants organiques non volatils tels que :
- les alcools aromatiques non volatils tels que l'alcool benzylique, le phenoxyéthanol ;
- les esters d'acides en C₁-C₂₀ liquides et d'alcools en C₁-C₈ non volatils tels que le myristate d'isopropyle ;
- l'éthylène carbonate, le propylène carbonate; le butylène carbonate ;
- les polyols non volatils tels que le glycérol, l'éthylène glycol, le dipropylène glycol, le butylène glycol ;
- les éthers de glycol non volatils comme le monoéthyléther de diéthylène glycol, le mono n-butyl éther de dipropylène glycol ;
- les huiles hydrocarbonées non volatiles telles que l'isohexadécane ;
- les alcools gras liquides non volatils en C₁₀-C₃₀ tels que l'alcool oléique, les esters d'alcools gras en C₁₀-C₃₀ liquides tels que les benzoates d'alcool gras en C₁₀-C₃₀ et leurs mélanges ; l'huile de polybutène, l'isononanoate d'isononyle, le malate d'isostéaryle, le tétra-isostéarate de pentaérythrityle, le trimélate de tridécyle ;
- les solvants perfluorés non volatils tels que le perfluoroperhydrophenanthrene, vendu sous la dénomination de "Flutec PC11®" par la Société F2 Chemicals.
- les silicones non volatiles de faible viscosité telles que la poly dimethylsiloxane de viscosité 5 cst commercialisée par Do Corning sous la dénomination Dow Corning 200 Fluid 5 cst et la poly dimethylsiloxane de viscosité 10 cst commercialisée par Do Corning sous la dénomination Dow Corning 200 Fluid 10 cst.

### Composés siliconés additionnels

Afin d'obtenir un meilleur étalement de la composition de l'invention ainsi qu'un gainage amélioré, la composition de l'invention peut aussi contenir un ou plusieurs polysiloxanes présentant une viscosité supérieure à 100 cst, préférentiellement supérieure à 300 cst. La viscosité de ces polysiloxanes peut être mesurée selon la norme ASTM D-445. De tels polysiloxanes peuvent être des huiles, des gommes ou des résines de silicone, les silicones réticulées.

A titre de polysiloxanes de viscosité supérieure à 100 cst, on peut notamment citer les polydiméthylsiloxanes; les alkyldiméthicones; les polyphénylméthylsiloxanes tels que les phényldiméthicones, les phényltriméthicones, et les vinylméthylméthicones; ainsi que les silicones modifiées par des groupements aliphatiques et / ou aromatiques, éventuellement fluorés, ou par des groupements fonctionnels tels que des groupements hydroxyles, thiols et / ou amines.

De tels polysiloxanes peuvent choisis parmi les silicones de formule (I) : 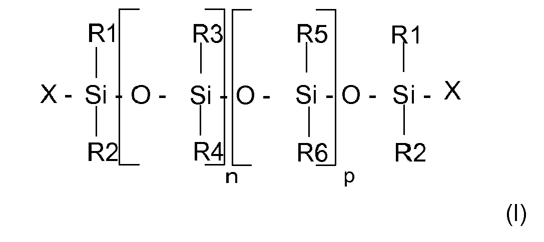 dans laquelle :
R₁, R₂, R₅ et R₆ sont, ensemble ou séparément, un radical alkyle ayant 1 à 6 atomes de carbone, R₃ et R₄ sont, ensemble ou séparément, un radical alkyle ayant de 1 à 6 atomes de carbone, un radical vinyle, un radical aryle, un radical aminoalkyle ayant de 1 à 6 atomes de carbone éventuellement substitué, un radical hydroxyle, un radical thioalkyle ayant de 1 à 6 atomes de carbone, X est un radical alkyle ayant de 1 à 6 atomes de carbone, un radical hydroxyle, un radical vinyle, un radical aminoalkyle ayant de 1 à 6 atomes de carbone éventuellement substitué, un radical thioalkyle ayant de 1 à 6 atomes de carbone, n et p étant des entiers choisis de manière à obtenir une viscosité supérieure à 300 cst.

A titre d'exemple, on peut citer les polydiméthylsiloxanes suivantes :
- les substituants R₁ à R₆ et X représentent un groupement méthyle, comme celle vendue sous la dénomination Baysilicone TP 3898 par la société Général Electric, et celle vendue sous la dénomination AK 500000 par la société Waker,
- les substituants R₁ à R₆ et X représentent un groupement méthyle, p et n sont tels que le poids moléculaire est de 120 000 g/mol, comme celle vendue sous la dénomination Dow Corning 200 Fluid 60000 CS par la société Dow Corning,
- les substituants R₁ à R₆ et X représentent un groupement méthyle, p et n sont tels que le poids moléculaire est de 250 000 g/mol, comme celle vendue sous la dénomination Mirasil DM 500.000 par la société Rhodia et celle vendue sous la dénomination Dow Corning 200 Fluid 500.000 cst par la société Dow Corning,
- les substituants R₁ à R₆ représentent un groupement méthyle, le groupement X représente un groupement hydroxy, n et p sont tels que le poids moléculaire du polymère soit de 600 000 g/mol, comme celle vendue sous la dénomination SGM 36 par la société Dow Corning,
- les diméthicones du type (polydiméthylsiloxane)(méthylvinylsiloxane) telle que la SE63 commercialisée par GE BAYER Silicones, les copolymères poly(diméthylsiloxane)(diphényl)(méthylvinylsiloxane), et leurs mélanges.

Dans le cas où le polysiloxane comprend un groupement fluoré, on peut choisir les copolymères de structure suivante : 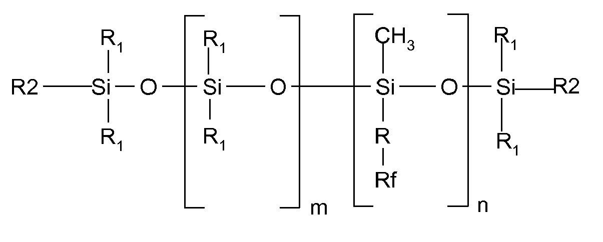 dans laquelle :
R représente un groupement divalent alkyle linéaire ou ramifié, ayant 1 à 6 atomes de carbone, de préférence un groupement divalent méthyle, éthyle, propyle ou butyle, Rf représente un radical fluoroalkyle, notamment un radical perfluoroalkyle, ayant 1 à 12 atomes de carbone, de préférence 1 à 9 atomes de carbone, R₁ représente, indépendamment l'un de l'autre, un radical alkyle en C₁-C₂₀, un radical hydroxyle, un radical phényle, R₂ représente R₁ ou R_{f},
m est choisi de 0 à 500, de préférence de 0 à 200, et n est choisi de 1 à 1000, de préférence de 1 à 500.

De préférence, les groupements R1 sont identiques et représentent un radical méthyle.

De tels polysiloxanes sont notamment ceux commercialisés par la société Shin Etsu sous les dénominations 'FL-5', 'FL-10', 'X22-821' et 'X22-822' ou encore 'FL-100', par la société Dow Corning sous le nom FS-1265 Fluid, par la société Phoenix Chemical sous la gamme Pecosil FS sous les dénominations Pecosil FSL-150, Pecosil FSL-300, Pecosil FSH-150, Pecosil FSH-300, Pecosil FSU-150, Pecosil FSU-300.

La masse moléculaire en poids du ou des polysiloxane peut être comprise entre 1000 et 1 500 000 g/mol, notamment entre 20 000 et 1 000 000 g/mol.

Le polysiloxane peut être sous forme de résine. Par le terme « résine », on entend une structure tridimensionnelle réticulée ou non. A titre d'exemple de résine de polysiloxane, on peut citer les silsesquioxanes et les siloxysilicates.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule (CH₃)₃SiO_{1/2}, l'atome de silicium étant relié à un seul atome d'oxygène dans le polymère comprenant cette unité.

La lettre D signifie une unité difonctionnelle (CH₃)SiO_{2/2} dans laquelle l'atome de silicium est relié à deux atomes d'oxygène

La lettre T représente une unité trifonctionnelle de formule (CH₃)SiO_{3/2}.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle SiO_{4/2} dans laquelle l'atome de silicium est lié à quatre atomes d'hydrogène eux mêmes liés au reste du polymère.

Divers résines de propriétés différentes peuvent être obtenues à partir de ces différentes unités, les propriétés des ces polymères variant en fonction du type de monomères (ou unités), du type et du nombre de radicaux subsitués, de la longueur de la chaîne polymérique, du degré de ramification et de la taille des chaines pendantes.

A titre d'exemple de ces résines silicones, on peut citer :
- les siloxysilicates qui peuvent être des triméthylsiloxysilicate de formule [(CH₃)₃SiO]ₓ(SiO_{4/2})_{y} (unités MQ) dans laquelle x et y sont des entiers allant de 50 à 80,
- les polysilesquioxanes de formule (CH₃SiO_{3/2})ₓ (unités T) dans laquelle au moins un des radicaux méthyle peut être substitué par un groupement R tel que défini plus haut. De préférence le nombre x d'unités T du silsesquioxane est inférieur ou égal à 500, il est plus préférentiellement compris entre 50 et 500. Le poids moléculaire de la résine de silicone selon l'invention est donc de préférence compris entre 500 et 50 000 g/mol, plus préférentiellement compris entre 500 et 20 000 g/mol et encore plus préférentiellement compris entre 500 et 10 000 g/mol ;
- les polyméthylsilsesquioxanes qui sont des polysilsesquioxanes dans lesquels aucun des radicaux méthyle n'est substitué par un autre groupement. De tels polymethylsilsesquioxanes sont décrits dans le document US 5 246 694 dont le contenu est incorporé par référence ;
- les polypropylsilsesquioxanes, pour lesquelles les radicaux méthyle sont remplacés par des radicaux propyle. Ces composés ainsi que leur synthèse sont notamment décrit dans la demande de brevet WO 2005/075567 ;
- les polyphénylsilsesquioxanes, pour lesquelles les radicaux méthyle sont remplacés par des radicaux phényle. Ces composés ainsi que leur synthèse sont notamment décrit dans la demande de brevet US 2004/0180011.

A titre d'exemples de résines polyméthylsilsesquioxanes commercialement disponibles, on peut citer celles qui sont commercialisées :
- par la société Wacker sous la référence Resin MK tels que la Belsil PMS MK : polymère comprenant des unités répétitives CH₃SiO_{3/2} (unités T), pouvant aussi comprendre jusqu'à 1 % en poids d'unités (CH₃)₂SiO_{2/2} (unités D) et présentant un poids moléculaire moyen d'environ 10 000 g/mol. On pense que le polymère se trouve dans une configuration « cage » et « échelle » comme cela est représenté dans les figures ci-dessous. Le poids moléculaire moyen des unités en configuration « cage » a été calculé à 536 g/mol. La majorité du polymère se trouve en configuration « échelle » avec des groupes éthoxy aux extrémités. Ces groupes éthoxy représentent 4,5 % en masse du polymère. Comme ces extrémités peuvent réagir avec l'eau, un taux faible et variable de groupes SiOH peut également être présent. 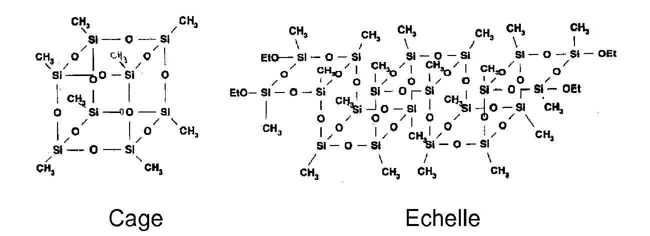
- par la société SHIN-ETSU sous les références KR-220L qui sont composées d'unités T de formule CH₃SiO_{3/2} et ont des groupes terminaux Si-OH (silanol), sous la référence KR-242A qui comprennent 98 % d'unités T et 2 % d'unités diméthyle D et ont des groupes terminaux Si-OH ou encore sous la référence KR-251 comprenant 88 % d'unités T et 12 % d'unités diméthyle D et ont des groupes terminaux Si-OH.

A titre d'exemples de résines polypropylsilsesquioxanes commercialement disponibles, on peut citer celles qui sont commercialisées :
- par la société DOW CORNING sous la référence Dow Corning 670 Fluid qui est une polypropylsilsesquioxane diluée dans la D5.

A titre d'exemples de résines polyphénylsilsesquioxanes commercialement disponibles, on peut citer celles qui sont commercialisées :
- par la société DOW CORNING sous la référence Dow Corning 217 Flake Resin qui est une polyphénylsilsesquioxane aux extrémités silanol ;
- par la société WACKER sous la référence Belsil SPR 45 VP.

Comme résines siloxysilicates, on peut citer les résines triméthylsiloxysilicate (TMS) éventuellement sous forme de poudres. De telles résines sont commercialisées sous la référence SR1000 par la société General Electric ou sous la référence TMS 803 par la société Wacker. On peut encore citer les résines timéthylsiloxysilicate commercialisées dans un solvant tel que la cyclométhicone, vendues sous la dénomination "KF-7312J" par la société Shin-Etsu, "DC 749", "DC 593" par la société Dow Corning.

La résine de silicone selon l'invention est de préférence filmogène. En effet, tous les silsesquioxanes ne sont pas filmogènes, par exemple les polyméthylsilsesquioxanes hautement polymérisées comme les TospearlTM de Toshiba ou la KMP590 de Shin-Etsu sont insolubles et ne sont pas filmogènes.

Dans un mode de réalisation de l'invention, la ou les résines de silicones sont solubles ou dispersables dans la composition de l'invention. De préférence, les résines de silicone selon l'invention sont solubles dans les silicones volatiles et les solvants organiques. Dans un mode de réalisation, la résine de silicone est solide à 25 °C.

Les résines de silicones préférées selon l'invention sont les résines triméthylsiloxysilicates, les résines polyméthylsilsesquioxanes et les résines polypropylsilsesquioxanes.

La composition de l'invention peut aussi contenir une silicone réticulée telle qu'un organopolysiloxane élastomère réticulé, un composé siliconé de haut poids moléculaire présentant une structure tridimensionnelle, aux propriétés viscoélastiques d'un matériau solide souple. Ces organopolysiloxanes peuvent ainsi se présenter sous forme sèche en poudre, ou sous forme gonflée, dans un solvant, le produit résultant étant généralement un gel. Ces produits peuvent également se présenter sous forme dispersée dans une solution aqueuse.

La synthèse de ces organopolysiloxane est décrite dans les brevets suivants :
- US 5 266 321 de Kobayashi Kose,
- US 4 742 142 de Toray Silicone,
- US 5 654 362 de Dow Corning Corp,
- la demande de brevet FR 2 864 784.

Les organopolysiloxanes élastomères utilisés dans la composition peuvent être partiellement ou totalement réticulés. Ils se présentent généralement sous forme de particules. En particulier, les particules d'organopolysiloxane élastomère ont une taille moyenne en nombre allant de 0,1 à 500 µm,. Ces particules peuvent avoir toute forme et par exemple être sphériques, plates ou amorphes.

L'organopolysiloxane réticulé obtenu peut être un composé non-émulsionnant ou un composé émulsionnant. Le terme "non émulsionnant" défini des organopolysiloxanes réticulés ne contenant pas de motifs polyoxyalkylène. Le terme "émulsionnant" signifie des composés organopoysiloxanes réticulés ayant au moins un motif polyoxyalkylène, notamment polyoxyéthylène ou polyoxypropylène.

Les particules d'organopolysiloxane réticulé peuvent être véhiculées sous forme de gel constitué d'un organopolysiloxane réticulé inclus dans au moins une huile hydrocarbonée et / ou une huile siliconée. Dans ces gels, les particules d'organopolysiloxane sont souvent des particules non-sphériques. Les particules d'organopolysiloxane réticulé peuvent également se présenter sous forme de poudre, notamment sous forme de poudre sphérique.

Des organopolysiloxanes réticulés non-émulsionnants sont notamment décrits dans les brevets US 4 970 252, US 4 987 169, US 5 412 004, US 5 654 362, US 5 760 116, dans la demande JP-A-61-194009.

Comme organopolysiloxanes réticulés non-émulsionnants, on peut utiliser ceux vendus sous les dénominations "KSG-6", "KSG-15", "KSG-16", "KSG-18", "KSG-31", "KSG-32", "KSG-33", "KSG-41", "KSG-42", "KSG-43", "KSG-44" « USG-103 » par la société Shin Etsu, "DC 9040", "DC9041", "DC 9509", "DC9505", "DC 9506" « DC 9045 » par la société Dow Corning, "GRANSIL" par la société Grant Industries, "SFE 839" par la société General Electric.

Avantageusement, les organopolysiloxanes réticulés émulsionnants comprennent les organopolysiloxanes modifiés polyoxyalkylène formé à partir de composés divinyliques, en particulier des polysiloxanes ayant au moins deux groupes vinyliques, réagissant avec des liaisons Si-H d'un polysiloxane. Des organopolysiloxanes réticulés émulsionnants sont notamment décrits dans les brevets US 5 236 986, US 5 412 004, US 5 837 793, US 5 811 487.

Comme organopolysiloxanes réticulés émulsionnants, on peut utiliser ceux commercialisés sous les dénominations "KSG-21 ", "KSG-20", "KSG-30"par la société Shin Etsu, et "DC9010", "DC9011" par la société Dow Corning.

Les particules d'organopolysiloxane réticulé élastomère peuvent se présenter également sous forme de poudre d'organopolysiloxane réticulé élastomère enrobée de résine de silicone, notamment de résine silsesquioxane, comme décrit par exemple dans le brevet US 5 538 793.

De tels élastomères sont vendus sous les dénomination "KSP-100", "KSP-101 ", "KSP-102", "KSP-103", KSP-104", "KSP-105" par la société Shin Etsu.

De préférence l'organopolysiloxane réticulé est non-émulsionnant.

La composition de l'invention peut aussi contenir un polymère siliconé greffé. Dans le cadre de l'invention, on entend par polymère siliconé greffé, un polymère comprenant une portion polysiloxane et une portion constituée d'une chaîne organique non-siliconée, l'une des deux portions constituant la chaîne principale du polymère l'autre étant greffée sur ladite chaîne principale.

Les polymères siliconés greffés utilisés dans la composition cosmétique selon l'invention sont choisis préférentiellement dans le groupe constitué par les polymères à squelette organique non siliconé greffé par des monomères contenant un polysiloxane, les polymères à squelette polysiloxanique greffé par des monomères organiques non siliconés et leurs mélanges.

Les monomères organiques non siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane, conformes à l'invention, peuvent être choisis parmi ceux décrits dans les brevets US 4,693,935, US 4,728,571 et US 4,972,037 et les demandes de brevet EP-A-0 412 704, EP-A-0 412 707, EP-A-0 640 105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Le copolymère à squelette organique non-siliconé greffé par des monomères contenant un polysiloxane peut par exemple avoir la structure suivante :

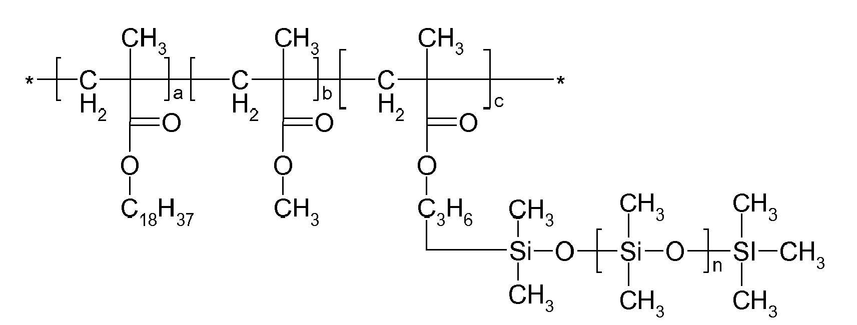

Un tel polymère est commercialisé sous le nom KP 561 commercialisés par Shin Etsu.

Le copolymère à squelette organique non-siliconé greffé par des monomères contenant un polysiloxane peut aussi avoir la structure suivante :

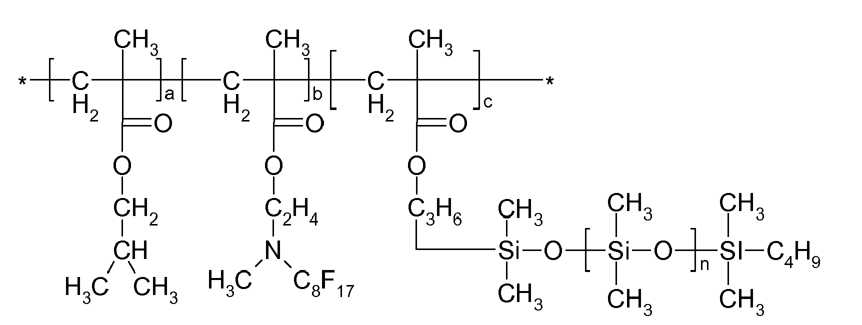

Un tel polymère, le Polysilicone 7, est commercialisé sous le nom SA70 par 3M.

D'autres copolymères à squelette organique non-siliconé greffé par des monomères contenant un polysiloxane peuvent aussi être le KP545, le KP574 et le KP575 commercialisés par Shin Etsu.

Comme composé siliconé greffé, on peut également citer le copolymère d'isobutylmethacrylate / bis-hydroxypropyl dimethicone acrylate commercialisé par Grant Industrie sous le nom Granacrysil BMAS.

Selon la présente invention, le ou les polymères siliconés greffés, à squelette polysiloxanique greffé par des monomères organiques non siliconés comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Des exemples de polymères siliconés correspondant à la définition sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle. Comme composé répondant à cette définition on peut citer le poly dimethyl / methyl siloxane à groupements propyl thio-3 acrylate de methyle / methacrylate de methyle / acide methacrylique ou Polysilicone-8 commercialisé sous le nom VS80 par la société 3M.

D'autres exemples de polymères siliconés sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non siliconés de l'invention varie de 10 000 à 1 000 000 environ, et encore plus préférentiellement de 10 000 à 100 000 environ.

De préférence, les polymères siliconés greffés sont choisis dans le groupe constitué par le copolymère de méthacrylate d'alkyles greffé polydiméthylsiloxane, les copolymères de méthacrylate d'isobutyle, d'acide acrylique et de macromère siliconé et le poly diméthyl / méthyl siloxane à groupements propyl thio-3-acrylate de méthyle / méthacrylate de méthyle / acide méthacrylique.

Selon un mode de réalisation particulier, la composition de l'invention comprend un ou plusieurs composés siliconés additionnels choisis parmi les polysiloxanes présentant une viscosité supérieure à 100 cst et les composés siliconés greffés.

Selon un mode de réalisation préféré, le ou les polysiloxanes présentant une viscosité supérieure à 100 cst sont choisis parmi les polydiméthylsiloxanes.

Selon un autre mode de réalisation préféré, le ou les polysiloxanes présentant une viscosité supérieure à 100 cst sont choisis parmi les résines de silicones.

Lorsqu'ils sont présents dans la composition conforme à l'invention, la quantité en composés siliconés additionnels est comprise entre 0,1 et 30 % en poids, de préférence entre 0,1 et 20 % en poids, et encore plus préférentiellement entre 0,1 et 10 % en poids du poids total de la composition.

### Autres additifs

Lorsque le polymère a une température de transition vitreuse trop élevée pour l'utilisation désirée, on peut y associer un plastifiant de façon à abaisser cette température du mélange utilisé. Le plastifiant peut être choisi parmi les plastifiants utilisés habituellement dans le domaine d'application, et notamment parmi les composés pouvant être des solvants pour le polymère.

De préférence, le plastifiant a une masse moléculaire inférieure ou égale à 5000 g/mol, de préférence inférieure ou égale à 2000 g/mol, préférentiellement inférieure ou égale à 1000 g/mol. Le plastifiant a avantageusement une masse moléculaire supérieure ou égale à 100 g/mol.

Ainsi, la composition peut comprendre, en outre, au moins un agent plastifiant. En particulier, on peut citer, seuls ou en mélange, les plastifiants usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les polyéthylène glycols, les polypropylène glycols, les copolymères polyéthylène glycols-polypropylène glycols et leurs mélanges, notamment les polypropylène glycols de haut poids moléculaire, ayant par exemple une masse moléculaire allant de 500 à 15000, tels que par exemple
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le dipropylène glycol butyléther. De tels composés sont commercialisés par Dow Chemical sous les dénominations Dowanol PPH et Dowanol DPnB.
- des esters d'acides, notamment carboxyliques, tels que des citrates, des phtalates, des adipates, des carbonates, de tartrates, des phosphates, des sébaçates ;
- les esters issus de la réaction d'un acide monocarboxylique de formule R₁₁COOH avec un diol de formule HOR₁₂OH avec R₁₁ et R₁₂, identiques ou différents, représentent une chaîne hydrocarbonée, comprenant de préférence de 3 à 15 atomes de carbone, linéaire, ramifiée ou cyclique, saturée ou insaturée comprenant éventuellement un ou plusieurs hétéroatomes tels que N, O , S, en particulier le monoester résultant de la réaction de l'acide isobutyrique et d'octanediol tel que le triméthyl-2,2,4-pentane-diol-1,3, tel que celui commercialisé sous la référence TEXANOL Ester Alcohol par la société Eastman Chemical,
- des dérivés oxyéthylénés, tels que les huiles oxyéthylénées, notamment les huiles végétales, telles que l'huile de ricin ;
- leurs mélanges.

Plus particulièrement, le plastifiant peut être choisi parmi les esters d'au moins un acide carboxylique comprenant 1 à 7 atomes de carbones et d'un polyol comprenant au moins 4 groupes hydroxyles.

Le polyol selon l'invention peut être un ose - polyhydroxyaldéhyde (aldose) ou polyhydroxycétone (cétose) - cyclisé ou non. Le polyol est de préférence un ose cyclisé sous forme d'hémi-acétal.

Le polyol peut être un mono- ou un polysaccharide comprenant de 1 à 10 oses, de préférence de 1 à 4, de préférence encore un ou deux oses. Le polyol peut être choisi parmi l'érythritol, le xylitol, le sorbitol, le glucose, le saccharose, le lactose, le maltose.

Le polyol selon l'invention est de préférence un disaccharide. Parmi les disaccharides, on peut citer le saccharose (appelé également alpha-D-glucopyranosyl-(1-2)-béta-D-fructofuranose), le lactose (appelé également béta-D-galactopyranosyl-(1-4)-béta-D-glucopyranose) et le maltose (appelé également alpha-D-glucopyranosyl-(1-4)-béta-D-glucopyranose), et de préférence le saccharose.

L'ester selon l'invention peut être constitué d'un polyol estérifié par au moins deux acides monocarboxyliques différents, ou par au moins trois acides monocarboxyliques différents.

L'ester selon l'invention peut être un copolymère de deux esters, en particulier un copolymère i) d'un saccharose substitué par des groupements benzoyle et ii) d'un saccharose substitué par des groupements acétyle et/ou isobutyryle.

L'acide carboxylique est de préférence un acide monocarboxylique comprenant de 1 à 7 atomes de carbones, de préférence de 1 à 5 atomes de carbone, par exemple choisi parmi les acides acétique, n-propanoïque, isopropanoïque, n-butanoïque, isobutanoïque, tertiobutanoïque, n-pentanoïque et benzoïque.

L'ester peut être obtenu à partir d'au moins deux acides monocarboxyliques différents. Selon un mode de mise en oeuvre, l'acide est un acide linéaire ou ramifié non substitué.

L'acide est de préférence choisi parmi l'acide acétique, l'acide isobutyrique, l'acide benzoïque, et leurs mélanges, et plus préférentiellement.

Selon un mode de mise en oeuvre préféré, l'ester est le di-acétate hexa-(2-méthyl-propanoate) de saccharose, tel que celui vendu sous la dénomination "Sustane SAIB Food Grade Kosher" par la société EASTMAN CHEMICAL.

Selon un autre mode de réalisation, le plastifiant peut être choisi parmi les esters d'acide polycarboxylique aliphatique ou aromatique et d'alcool aliphatique ou aromatique comprenant de 1 à 10 atomes de carbone.

L'alcool aliphatique ou aromatique comprend de 1 à 10 atomes de carbone, de préférence de 1 à 8, par exemple de 1 à 6. Il peut être choisis parmi les alcools R1OH, tels que R1 représente méthyle, éthyle, propyle, isopropyle, butyle, hexyle, éthylhexyle, décyle, isodécyle, benzyle, ou benzyle substitué par un alkyle comprenant 1 à 3 atomes de carbone, et leurs mélanges.

L'acide polycarboxylique aliphatique ou aromatique comprend de préférence de 3 à 12 atomes de carbone, de préférence de 3 à 10 atomes de carbone, de préférence de 3 à 8 atomes de carbone, par exemple 6 ou 8 atomes de carbones.

L'acide polycarboxylique aliphatique ou aromatique est avantageusement choisi parmi les acides dicarboxyliques et les acides tricarboxyliques.

Parmi les acides dicarboxyliques aliphatiques, on peut citer ceux de formule HOOC-(CH2)n-COOH, dans laquelle n est un entier allant de 1 à 10, de préférence allant de 2 à 8, par exemple égal à 2, 4, 6 ou 8.

On préfère les acides dicarboxyliques choisis parmi l'acide succinique, l'acide adipique et l'acide sébacique.

Parmi les acides dicarboxyliques aromatiques, on peut citer l'acide phtalique.

Parmi les acides tricarboxyliques, on peut citer les triacides qui correspondent à la formule 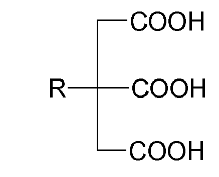 dans laquelle R représente un groupement -H, -OH ou -OCOR' dans lequel R' représente un groupement alkyle ayant de 1 à 6 atomes de carbone. De préférence, R représente un groupement -OCOCH₃.

L'acide tricarboxylique est notamment choisi parmi l'acide acétyl-citrique, l'acide butyroyl-citrique, l'acide citrique.

Parmi les esters d'acide tricarboxylique, on peut utiliser les esters dérivés de l'acide citrique (ou citrates) tels que l'acétyl-citrate de tributyle, l'acétyl-citrate de triéthyle, l'acétyl-citrate de triéthylhexyle, l'acétyl-citrate de trihexyle, le butyroyl- citrate de trihexyle, le citrate de triisodécyle, le citrate de triisopropyle, le citrate de tributyle et le citrate de tri(éthyl-2- hexyle). Comme références commerciales de plastifiants mentionnés ci-dessus on peut citer, la gamme Citroflex commercialisée par Vertellus avec notamment le Citroflex A4 et le Citroflex C2.

Parmi les esters de l'acide adipique, on peut citer l'adipate de dibutyle et l'adipate de di(éthyl-2-hexyle).

Parmi les esters de l'acide sébacique, on peut citer le sébaçate de dibutyle, le sébaçate de di(éthyl-2-hexyle), le sébaçate de diéthyle et le sébaçate de diisopropyle.

Parmi les esters de l'acide succinique, on peut citer le succinate de di(éthyl-2-hexyle) et le succinate de diéthyle.

Parmi les esters de l'acide phtalique, on peut citer le phtalate de butyle et de benzyle, le phtalate de dibutyle, le phtalate de diéthylhexyle, le phtalate de diéthyle et le phtalate de diméthyle.

Avantageusement, le plastifiant peut être présent dans la composition en une teneur telle que le rapport massique entre le polymère filmogène hydrophobe et le plastifiant est compris entre 0,5 et 100, de préférence entre 1 et 50, de préférence entre 1 et 20.

La composition selon l'invention peut comprendre un ou plusieurs agents épaississants choisis parmi les agents épaississants polymériques, les agents épaississants minéraux et leurs mélanges.

L'épaississant peut être minéral ou organique, polymère ou non polymère. L'épaississant peut être choisi pour épaissir une phase aqueuse ou une phase grasse de la composition selon les cas.

On entend par épaississant, un composé qui modifie la rhéologie du milieu dans lequel il est incorporé en accroissant d'au moins 100 cps la viscosité du milieu à 25 °C et à un taux de cisaillement de 1 s⁻¹. Cette viscosité peut être mesurée à l'aide d'un viscosimètre cône/plan (Rhéomètre Haake R600 ou analogue).

L'épaississant de milieu aqueux peut être choisi parmi :
- les argiles hydrophiles,
- la silice pyrogénée hydrophile,
- les épaississants cellulosiques hydrosolubles, tels que l'hydroxyethylcellulose, la méthylcellulose, l'hydroxypropylcellulose. Parmi celles-ci on peut citer notamment les gommes vendues sous la dénomination Cellolize QP 4400 H par a société Amercol.
- les gommes de guar non ionique comprenant des groupements hydroxyalkyle en C₁-C₆. On peut mentionner à titre d'exemple les groupements hydroxymethyle, hydroxypropyle et hydroxybutyle. De telles gommes de guar sont notamment vendue sous les dénominations commerciales JAGUAR HP8, JAGUAR HP60, JAGUAR HP120, JAGUAR HP105 par la société MEYHALL ou sous la dénomination GALACTASOL 40H4FD2 par la société AQUALON.
- les carraghénanes,
- les gommes de caroube, de scléroglucane, de gellane, de rhamsan, de karaya,
- les alginates, les maltodextrines, l'amidon et ses dérivés, l'acide hyaluronique et ses sels,
- les polymères poly(métha)crylates de glycéryle vendus sous les dénominations de "Hispagel" ou "Lubragel" par les Sociétés Hispano Quimica ou Guardian,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic par la Société Allied Colloïd, ou encore,
- les homopolymères réticulés de chlorure de méthacryloyloxyéthyltriméthylammonium vendus sous la dénomination de "Salcare SC95" par la Société Allied Colloïd,
- les polymères associatifs et notamment les polyuréthanes associatifs.

De tels épaississants sont notamment décrits dans les demandes EP-A-1400234, dont le contenu est incorporé à titre de référence.

L'épaississant de milieu huileux peut être choisi parmi
- les argiles organophiles,
- les silices pyrogénées hydrophobes
- les gommes de guar alkylées (avec groupe alkyle en C1-C6), telles que celles décrites dans EP-A-708114 ;
- les polymères gélifiant d'huile comme les polymères tribloc ou en étoile résultant de la polymérisation ou copolymérisation d'au moins monomère à groupe éthylénique, comme les polymères vendus sous la dénomination Kraton ;
- les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et / ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619 dont le contenu est incorporé à titre de référence ; en particulier les résines de polyamides (notamment comprenant des groupes alkyles ayant de 12 à 22 atomes de carbone) telles que celles décrites dans US-A-5783657 dont le contenu est incorporé à titre de référence.
- les résines de polyamides siliconées telles que décrites dans la demande EP-A-1266647, dans la demande de brevet français déposée sous le n° 0 216 039 dont le contenu est incorporé à titre de référence.

De tels épaississants sont notamment décrits dans les demandes EP-A-1400234, dont le contenu est incorporé à titre de référence.

L'épaississant peut être un agent gélifiant organique, c'est-à-dire un agent comprenant au moins un composé organique. Les organogélateurs peuvent être choisis parmi ceux décrits dans la demande WO-A-03/105788 dont le contenu est incorporé à titre de référence.

Plus précisément, l'agent épaississant polymérique présent dans la composition selon l'invention est un polymère amorphe formé par polymérisation d'une oléfine. L'oléfine peut être notamment un monomère à insaturation éthylénique élastomérique.

Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

L'agent épaississant polymérique est capable d'épaissir ou de gélifier la composition. Par polymère amorphe, on entend un polymère qui n'a pas de forme cristalline. L'agent épaississant polymérique peut être également filmogène.

L'agent épaississant polymérique peut être notamment un copolymère dibloc, tribloc, multibloc, radial, étoile, ou leurs mélanges.

De tels agents épaississants polymériques sont décrits dans la demande US-A-2002/005562 et dans le brevet US-A-5 221 534

Avantageusement, l'agent épaississant polymérique est un copolymère bloc amorphe de styrène et d'oléfine.

L'agent épaississant polymérique est de préférence hydrogéné pour réduire les insaturations éthyléniques résiduelles après la polymérisation des monomères.

En particulier, l'agent épaississant polymérique est un copolymère, éventuellement hydrogéné, à blocs styrène et à blocs éthylène/alkylène en C3-C4.

Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

On peut également utiliser un mélange de copolymère hydrogéné tribloc styrène-butylène / éthylène-styrène et de polymère étoile hydrogéné éthylènepropylène-styrène, un tel mélange étant notamment dans l'isododécane. De tels mélanges sont par exemple vendus par la société PENRECO sous les dénominations commerciales VERSAGEL® M5960 et VERSAGEL® M5670.

Avantageusement, on utilise comme agent épaississant polymérique un copolymère dibloc tel que ceux décrits précédemment, en particulier un copolymère dibloc de styrène-éthylène / propylène.

Plus précisément, les argiles organophiles sont des argiles modifiées par des composés chimiques rendant l'argile apte à gonfler.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, on utilise comme agent épaississant minéral une hectorite ou une bentonite organomodifiée.

Le ou les agents épaississants peuvent être présents dans la composition en une teneur totale allant de 0,1 à 10 % en poids par rapport au poids total de la composition, de préférence allant de 0,5 à 7 % en poids, et plus préférentiellement allant de 0,5 à 4 % en poids.

### Composés additionnels

La composition conforme à l'invention peut également contenir au moins un agent utilisé habituellement en cosmétique, choisi, par exemple, parmi des agents réducteurs, des corps gras, des adoucissants, des agents anti-mousse, des agents hydratants, des filtres UV, des colloïdes minéraux, des peptisants, des parfums, des tensio-actifs anioniques, cationiques, non ioniques ou amphotères, des protéines, des vitamines, des propulseurs, les cires oxyéthylénées ou non, les paraffines, les acides gras en C₁₀-C₃₀ tels que l'acide stéarique, l'acide laurique, les amides gras en C₁₀-C₃₀ tel que le diéthanolamide laurique, des polymères anioniques, cationiques, non ioniques ou amphotères.

Les additifs ci-dessus sont en général présents en quantité comprise pour chacun d'eux entre 0,01 et 20 % en poids par rapport au poids de la composition.

Bien entendu, l'homme de l'art veillera à choisir ce ou ces éventuels additifs de manière telle que les propriétés avantageuses attachées intrinsèquement à la formation du gainage conforme à l'invention ne soient pas, ou substantiellement pas, altérées.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de mousse, de stick, de dispersion de vésicules notamment de lipides ioniques ou non, de lotion biphase ou multiphase, de spray, de poudre, de pâte. La composition peut également se présenter sous forme de laque.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition peut être une composition anhydre, c'est-à-dire une composition contenant moins de 2 % en poids d'eau, voire moins de 0,5 % d'eau, notamment exempte d'eau, l'eau n'étant pas ajoutée lors de la préparation de la composition mais correspondant à l'eau résiduelle apportée par les ingrédients mélangés.

Selon un mode de réalisation particulier, la composition de l'invention est anhydre.

La composition décrite ci-dessus peut être mise en oeuvre sur cheveux secs ou humides ainsi que sur tous types de cheveux clairs ou foncés, naturels ou colorés, permanentés, décolorés ou défrisés.

Selon un mode de réalisation particulier du procédé de l'invention, les cheveux sont lavés avant application de la composition décrite ci-dessus. De préférence, l'application de la composition se fait sur cheveux propres.

L'application peut se faire sur des cheveux secs ou humides.

L'application sur les cheveux peut être mise en oeuvre par exemple au moyen d'un peigne, d'un pinceau à l'aide d'une brosse ou aux doigts.

Selon un mode de réalisation particulier de l'invention, l'application de la composition est ensuite suivie d'un séchage à une température supérieure à 40 °C. De préférence, cette température est supérieure à 45 °C. Encore plus préférentiellement, cette température est supérieure à 45 °C et inférieure à 220 °C.

Le séchage peut être réalisé immédiatement après l'application ou après un temps de pose pouvant aller de 1 minute à 30 minutes.

De préférence les cheveux sont séchés, en plus d'un apport de chaleur, avec un flux d'air. Ce flux d'air pendant le séchage permet d'améliorer l'individualisation du gainage.

Durant le séchage, une action mécanique sur les mèches peut être exercée telle qu'un peignage, un brossage, le passage des doigts.

L'étape de séchage du procédé de l'invention peut être mise en oeuvre avec un casque, un sèche-cheveux, un fer à lisser, un climazon, ...

Lorsque l'étape de séchage est mise en oeuvre avec un casque ou un sèche-cheveux, la température du séchage est comprise entre 40 et 110 degrés, de préférence entre 50 et 90 degrés.

Lorsque l'étape de séchage est mise en oeuvre avec un fer à lisser, la température du séchage est comprise entre 110 et 220 degrés, de préférence entre 140 et 200 degrés.

Une fois le séchage terminé, un rinçage ou un shampooing terminal peut éventuellement être réalisé.

### EXEMPLES

### Exemple 1

Les compositions suivantes sont réalisées :

| Composition | 1 a | 1 b | 1 c | 1 d | 1 e |
|---|---|---|---|---|---|
| Polymère supramoléculaire 1 a obtenu à partir de GI3000 à titre de composé A et d'un greffon de formule B dans lequel L désigne un radical hexaméthylène | 8 g | - | - | - | - |
| Polymère supramoléculaire 1 b obtenu à partir de GI3000 à titre de composé A et d'un greffon de formule B dans lequel L désigne un radical isophorone | - | 8 g | - | - | - |
| Polymère supramoléculaire 1 c obtenu à partir de GI3000 à titre de composé A et d'un greffon de formule B dans lequel L désigne un radical 4,4'-dicyclohexylméthane | - | - | 8g | - | - |
| Polymère supramoléculaire 1 d obtenu à partir de GI2000 à titre de composé A et d'un greffon de formule B dans lequel L désigne un radical 4,4'-dicyclohexylméthane | - | - | - | 8 g | - |
| Polymère supramoléculaire 1 e obtenu à partir de GI2000 à titre de composé A et d'un greffon de formule B dans lequel L désigne un radical isophorone | - | - | - | - | 8 g |
| Mélange Polydimethylsiloxane alpha-omega dihydroxyle / Cyclopentadimethylsiloxane (14,7 / 85,3) commercialisé par Dow Corning sous le nom DC1501 Fluid (*) | 14 g | 14 g | 14 g | 14 g | 14 g |
| Polyméthylsilsesquioxane commercialisé sous le nom Wacker Belsil PMS MK Powder par la société Wacker | 3 g | 3 g | 3 g | 3 g | 3 g |
| Nacre mica enrobé d'oxyde de fer brun commercialisée par Eckart sous le nom Prestige Bronze | 6 g | 6 g | 6 g | 6 g | 6 g |
| Disteardimonium hectorite (10 %) et propylene carbonate (3 %) dans l'isododécane commercialisé par Elementis sous le nom Bentone Gel ISD V | 8 g | 8 g | 8 g | 8 g | 8 g |
| Isododécane | qs 100 g | qs 100 g | qs 100 g | qs 100 g | qs 100 g |

| | | | | | |
|---|---|---|---|---|---|
| * les concentrations indiquées correspondent au polymère pur. | | | | | |

0,6 g de la composition 1 a, 1b, 1 c, 1 d ou 1 e est appliqué sur une mèche de 1 g de cheveux de hauteur de ton 4 propres et secs. Après 2 minutes de pause, la mèche est séchée au sèche-cheveux à une température de 80 °C pendant 2 minutes. On obtient une mèche colorée dont les cheveux sont individualisés et dont la couleur est très homogène et rémanente au shampooing.

### Exemple 2

La composition suivante est réalisée :

| Composition | 2 |
|---|---|
| Polymère supramoléculaire 1a obtenu à partir de GI3000 à titre de composé A et d'un greffon de formule B dans lequel L désigne un radical hexaméthylène | 4 g |
| Mélange Polydimethylsiloxane alpha-omega dihydroxyle / Cyclopentadimethylsiloxane (14,7 / 85,3) commercialisé par Dow Corning sous le nom DC1501 Fluid (*) | 14 g |
| Nacre mica enrobé d'oxyde de fer brun commercialisée par Eckart sous le nom Prestige Bronze | 2 g |
| Ethanol | 5 g |
| Isododécane | qs 100 g |

| | |
|---|---|
| * la concentration indiquée correspond au polymère pur. | |

0,6 g de la composition 2 est appliqué sur une mèche de 1 g de cheveux de hauteur de ton 4 propres et humides. Après 2 minutes de pause, la mèche est séchée au sèche-cheveux à une température de 80 °C pendant 2 minutes. On obtient une mèche colorée dont les cheveux sont individualisés et dont la couleur est très homogène et rémanente au shampooing.

### Exemple 3

La composition suivante est réalisée :

| Composition | 1a |
|---|---|
| Polymère supramoléculaire 1a obtenu à partir de GI3000 à titre de composé A et d'un greffon de formule B dans lequel L désigne un radical hexaméthylène | 8 g |
| Mélange Polydimethylsiloxane alpha-omega dihydroxyle / Cyclopentadimethylsiloxane (14,7 / 85,3) commercialisé par Dow Corning sous le nom DC1501 Fluid (*) | 14 g |
| Polyméthylsilsesquioxane commercialisé sous le nom Wacker Belsil PMS MK Powder par la société Wacker | 3 g |
| Nacre mica enrobé d'oxyde de fer brun commercialisée par Eckart sous le nom Prestige Bronze | 6 g |
| Disteardimonium hectorite (10%) et propylene carbonate (3%) dans l'isododécane commercialisé par Elementis sous le nom Bentone Gel ISD V | 8 g |
| Isododécane | qs 100 g |

| | |
|---|---|
| * la concentration indiquée correspond au polymère pur. | |

0,6 g de la composition 1 a est appliqué sur une mèche de 1 g de cheveux de hauteur de ton 4 propres et secs. Après 2 minutes de pause, la mèche est séchée avec un fer à lisser à 130 °C pendant une minute. La mèche est ensuite peignée. On obtient une mèche colorée dont les cheveux sont individualisés et dont la couleur est très homogène et rémanente au shampooing.

### Exemple 4

La composition suivante est réalisée :

| Composition | 3 |
|---|---|
| Polymère supramoléculaire 1a obtenu à partir de GI3000 à titre de composé A et d'un greffon de formule B dans lequel L désigne un radical hexaméthylène | 8 g |
| Mélange Polydimethylsiloxane alpha-omega dihydroxyle / Cyclopentadimethylsiloxane (14,7 / 85,3) commercialisé par Dow Corning sous le nom DC1501 Fluid (*) | 14 g |
| Polyméthylsilsesquioxane commercialisé sous le nom Wacker Belsil PMS MK Powder par la société Wacker | 3 g |
| Résine Triméthyl siloxysilicate commercialisée sous le nom SR1000 par Momentive Performance materials | 2 g |
| Nacre mica enrobé d'oxyde de fer brun commercialisée par Eckart sous le nom Prestige Bronze | 6 g |
| Disteardimonium hectorite (10%) et propylene carbonate (3%) dans l'isododécane commercialisé par Elementis sous le nom Bentone Gel ISD V | 8 g |
| Isododécane | qs 100 g |

| | |
|---|---|
| * la concentration indiquée correspond au polymère pur. | |

0,6 g de la composition 3 est appliqué sur une mèche de 1 g de cheveux de hauteur de ton 4 propres et humides. Après 2 minutes de pause, la mèche est séchée au sèche-cheveux à une température de 80 °C pendant 2 minutes. On obtient une mèche colorée dont les cheveux sont individualisés et dont la couleur est très homogène et rémanente au shampooing.

Les polymères supramoléculaires utilisés dans les exemples ci-dessus sont synthétisés de la manière suivante.

Polymère 1a : 100 g de polymère de GI3000 commercialisé par la société NISSO sont séchés à 80 °C, sous vide pendant une nuit. Ce polymère est mis en solution dans 400 mL de toluène anhydre. 25 µL de catalyseur, le dilaurate de dibutyl étain sont additionnés au mélange réactionnel. Le milieu est chauffé à 80 °C, et mélangé jusqu'à obtenir une solutuion homogène. 15 g de molécule fonctionnalisée isocyanate de structure suivante : 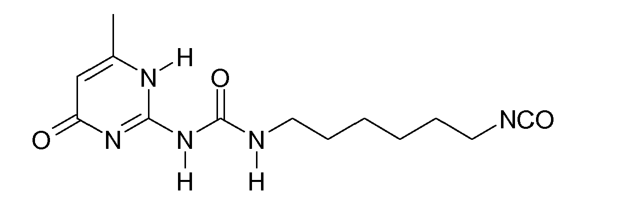 sont additionnés en solution dans 300 mL de toluène anhydre, sous atmosphère contrôlée à 40 °C. Le mélange réactionnel est chauffé à 100 °C et agité à cette température pendant 4 heures . Le suivi de la réaction est fait par spectroscopie infra rouge, avec le suivi de la disparition totale du pic caractéristique des isocyanate à 2260 cm⁻¹. A la fin de la réaction, 100 mL d'éthanol sont ajoutés pour éliminer toute trace d'isocyanate résiduel. Le mélange est filtré après avoir rajouté de l'isododécane pour rendre la solution moins visqueuse. La solution de polymère est alors directement strippée à l'isododécane. Le polymère final est obtenu à 21 % d'extrait sec dans l'isododécane et est caractérisé par GPC (Mn = 6400 et un indice de polydispersité Ip de 1,85) et RMN ¹H (spectre conforme à ce qui est attendu).

Polymère 1b : 99 g de polymère GI3000 commercialisé par la société NISSO, en présence de 22 mg de catalyseur, le dilaurate de dibutyl étain, sont chauffés à 80 °C, sous vide pendant 2 heures. La température du mélange est descendue à 20 °C, sous argon. 11 g d'IPDI (isophorone diisocyanate) sont additionnés, ainsi que 30 mL d'isododécane. Le mélange est agité pendant 16 heures à 20 °C, sous atmosphère contrôlée, puis est chauffé à 120 °C, suivi de l'addition de 25 mL de propylène carbonate. 8,1 g de 6-méthyl isocytosine sont additionnés. Il en résulte une suspension homogène blanche. Cette suspension est chauffée à 140 °C et agitée à cette température pendant 6 heures. La réaction est suivie par spectroscopie infra-rouge, jusqu'à la disparition totale du pic caractériistique des isocyanates (2250 cm⁻¹). Le mélange est ensuite redescendu à 30 °C, et 1 litre d'heptane est additionné au mélange, avant filtration sur célite. Un stripping à l'isododécane permet d'obtenir le polymère 1 b à 20 % d'extrait sec. Le polymère est caractérisé par GPC (Mn =4200 avec un Ip de 2,34).

Polymère 1c : 89 g de polymère GI3000 commercialisé par la société NISSO, en présence de 22 mg de catalyseur, le dilaurate de dibuylétain, sont chauffés à 80 °C, sous vide pendant 2 heures. La température du mélange est descendue à 20 °C, sous argon. 11,6 g de 4,4'-dicyclohexylméthane diisocyanate sont additionnés, suivi de l'addition de 60 mL d'isododécane. Le mélange est agité pendant 16 heures à 20 °C, sous atmosphère contrôlée, puis est chauffé à 120 °C, suivi de l'addition de 40 mL de propylène carbonate. 6,64 g de 6-méthyl isocytosine sont additionnés. Il en résulte une suspension homogène blanche. Cette suspension est chauffée à 140 °C et agitée à cette température pendant 8 heures. La réaction est suivie par spectroscopie infra-rouge, jusqu'à la disparition totale du pic caractériistique des isocyanates (2250 cm⁻¹). Le mélange est ensuite redescendu à 30 °C, et 250 mL d'isododécane ainsi que 500 mL d'heptane sont additionnés au mélange, avant filtration sur célite. Un stripping à l'isododécane permet d'obtenir le polymère 1c à 22 % d'extrait sec. Le polymère est caractérisé par GPC (Mn = 10700 avec un Ip de 2,26).

Polymère 1d : 143,1 g de polymère GI2000 commercialisé par la société NISSO, en présence de 33 mg de catalyseur, le dilaurate de dibutyl étain, sont chauffés à 80 °C, sous vide pendant 2 heures. La température du mélange est descendue à 20 °C, sous argon, suivi de l'addition de 85 mL d'isododécane. 30,8 g de 4,4'-dicyclohexylméthane diidocyanate sont additionnés. Le mélange est agité pendant 16 heures à 20 °C, sous atmosphère contrôlée, puis est chauffé à 120 °C, suivi de l'addition de 70 mL de propylène carbonate. 22,6 g de 6-méthyl isocytosine sont additionnés. Il en résulte une suspension homogène blanche. Cette suspension est chauffée à 140 °C et est agitée à cette température pendant 8 heures. La réaction est suivie par spectroscopie infra-rouge, jusqu'à la disparition totale du pic caractéristique des isocyanates (2250 cm⁻¹). Le mélange est ensuite redescendu à 20 °C, et 700 mL d'isododécane ainsi que 500 mL d'heptane sont additionnés au mélange, avant filtration sur célite. Un stripping à l'isododécane, permet d'obtenir le polymère 1 d à 20 % d'extrait sec. Le polymère est caractérisé par GPC (Mn = 8400 avec un Ip de 2,00).

Polymère 1e : 106,1 g de polymère GI2000 commercialisé par la société NISSO, en présence de 22 mg de catalyseur, le dilaurate de dibutyl étain, sont chauffés à 80 °C, sous vide pendant 2 heures. La température du mélange est descendue à 20 °C, sous argon, suivi de l'addition de 10 mL d'isododécane. 19,3 g d'isophorone diisocyanate sont additionnés. Le mélange est agité pendant 16 heures à 20 °C, sous atmosphère contrôlée, puis est chauffé à 120 °C, suivi de l'addition de 25 mL de propylène carbonate. 12 g de 6-méthyl isocytosine sont additionnés. Il en résulte une suspension homogène blanche. Cette suspension est chauffée à 140 °C et est agitée à cette température pendant 6 heures. La réaction est suivie par spectroscopie infra-rouge, jusqu'à la disparition totale du pic caractéristique des isocyanates (2250 cm⁻¹). Le mélange est ensuite redescendu à 30 °C, et 400 mL d'heptane, 200 mL de THF et 50 mL d'éthanol sont additionnés au mélange, avant filtration sur célite. Un stripping à l'isododécane, permet d'obtenir le polymère 1 e à 20 % d'extrait sec. Le polymère est caractérisé par GPC (Mn = 7000 avec un Ip de 2,05).

## Revendications

1. Composition de coloration des fibres kératiniques comprenant un ou plusieurs polymères supramoléculaires à base de polyalcène, un ou plusieurs pigments et un ou plusieurs solvants volatils, le rapport pondéral polymères supramoléculaires / pigments étant supérieur à 0,25.

2. Composition selon la revendication 1 dans laquelle le polyalcène est choisi parmi les poly(éthylènebutylène), les polybutadiènes et les polyisoprènes.

3. Composition selon les revendications 1 ou 2 dans laquelle le ou les polymères supramoléculaires à base de polyalcène sont issus de la condensation d'au moins un polymère polyalcène fonctionnalisé par au moins un groupe réactif, avec au moins un greffon fonctionnalisé par au moins un groupe réactif susceptible de réagir avec le ou les groupes réactifs du polymère poly(alcène) fonctionnalisé, le dit greffon étant porteur d'au moins un groupe capable de former au moins 3 liaisons H.

4. Composition selon la revendication 3 dans laquelle le polyalcène fonctionnalisé est de formule A :
HX-R-X'H
XH et X'H étant des groupes réactifs, avec X et X', identiques ou différents, choisis parmi O, S, NH, ou NRₐ, Rₐ représentant un groupe alkyle en C₁-C₆ ;
R représentant un homo ou un copolymère issu d'un ou plusieurs alcènes mono ou polyinsaturés en C₂-C₁₀.

5. Composition selon l'une quelconque des revendications 1 à 3 dans laquelle le polyalcène fonctionnalisé est choisi parmi les poly(éthylènebutylène) à extrémités hydroxyles, les polybutadiènes à extrémités hydroxyles, et les polyisoprènes à extrémités hydroxyles.

6. Composition selon l'une quelconque des revendications 3 à 4 dans laquelle le greffon possède au moins un groupe uréidopyrimidone.

7. Composition selon l'une quelconque des revendications 3 à 5 dans laquelle le ou les groupes réactifs du greffon sont des groupes isocyanates.

8. Composition selon l'une quelconque des revendications 3 à 6 dans laquelle le greffon est de formule (B) : 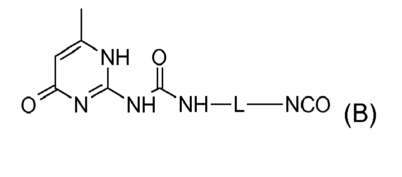 L désignant un groupement : phénylène; 1,4-nitrophényle ; 1,2-éthylène; 1,6-héxylène; 1,4-butylène; 1,6-(2,4,4-triméthylhexylène) ; 1,4-(4-méthylpentylène); 1,5-(5-méthylhexylène); 1,6-(6-méthylheptylène); 1,5-(2,2,5-triméthylhexylène) ; 1,7-(3,7-diméthyloctylène) ; -isophorone- ; 4,4'-méthylène biscyclohexylène; tolylène ; 2-méthyl-1,3-phénylène ; 4-méthyl-1,3-phénylène ; 4,4-biphénylèneméthylène.

9. Composition selon les revendications 1 ou 2 dans laquelle le ou les polymère supramoléculaires à base de polyalcène sont obtenus à partir d'un polymère (A1) comportant une partie polyalcène, le dit polymère étant fonctionnalisé par au moins un groupe réactif (B1), par condensation avec au moins une molécule (A3) comportant au moins un groupe réactif (B2), la dite molécule étant tel qu'après réaction des groupes (B1) et (B2) il y ait formation d'une entité capable de former au moins 3 liaisons H, de préférence au moins 4 liaisons H.

10. Composition selon la revendication 9 dans laquelle les polymères (A1) sont de formule (C1) :
CON-L-NCO-X-R-X'-CON-L-NCO (C1)
dans laquelle avec X et X', identiques ou différents, choisis parmi O, S, NH, ou NRₐ, Rₐ représentant un groupe alkyle en C₁-C₆ ;
R représentant un homo ou un copolymère issu d'un ou plusieurs alcènes mono ou polyinsaturés en C₂-C₁₀ ;
L désignant un groupement : phénylène; 1,4-nitrophényle ; 1,2-éthylène; 1,6-héxylène; 1,4-butylène; 1,6-(2,4,4-triméthylhexylène) ; 1,4-(4-méthylpentylène); 1,5-(5-méthylhexylène); 1,6-(6-méthylheptylène); 1,5-(2,2,5-triméthylhexylène) ; 1,7-(3,7-diméthyloctylène) ; -isophorone- ; 4,4'-méthylène biscyclohexylène; tolylène ; 2-méthyl-1,3-phénylène ; 4-méthyl-1,3-phénylène ; 4,4-biphénylèneméthylène.

11. Composition selon la revendication 9 dans laquelle la molécule (A3) est la 6 méthylisocytosine de formule

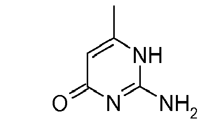

12. Composition selon l'une quelconque des revendications précédentes dans laquelle le polymère supramoléculaire de l'invention est de formule C : 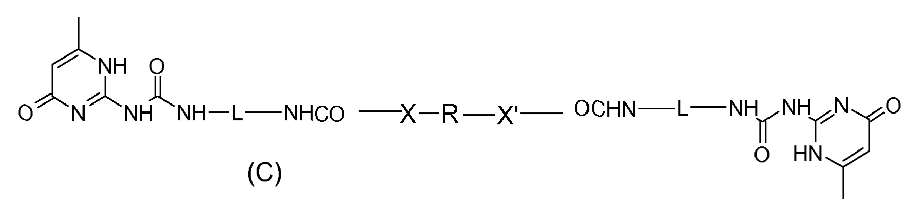 avec X et X', identiques ou différents, choisis parmi O, S, NH, ou NRₐ, Rₐ représentant un groupe alkyle en C₁-C₆;
R représentant un homo ou un copolymère issu d'un ou plusieurs alcènes mono ou polyinsaturés en C₂-C₁₀ ;
et L désignant un groupement : phénylène ; 1,4-nitrophényle; 1,2-éthylène; 1,6-héxylène; 1,4-butylène; 1,6-(2,4,4-triméthylhexylène) ; 1,4-(4-méthylpentylène); 1,5-(5-méthylhexylène) ; 1,6-(6-méthylheptylène) ; 1,5-(2,2,5-triméthylhexylène) ; 1,7-(3,7-diméthyloctylène) ; -isophorone- ; 4,4'-méthylène biscyclohexylène; tolylène ; 2-méthyl-1,3-phénylène; 4-méthyl-1,3-phénylène; 4,4-biphénylèneméthylène.

13. Composition selon l'une quelconque des revendications précédentes dans laquelle le ou les solvants volatils sont choisis parmi l'eau et les solvants organiques non siliconés et siliconés.

14. Composition selon l'une quelconque des revendications précédentes comprenant un ou plusieurs composés siliconés additionnels choisis parmi les polysiloxanes présentant une viscosité supérieure à 100 cst et les composés siliconés greffés.

15. Procédé de coloration des fibres kératiniques comprenant l'application sur les fibres kératiniques d'une composition telle que définie à l'une quelconque des revendications 1 à 14, éventuellement suivie d'un séchage à une température supérieure à 40 °C.

16. Utilisation d'une composition telle que définie à l'une quelconque des revendications 1 à 14 pour l'obtention de gainage coloré sur les cheveux.
